# EUROPEAN PATENT APPLICATION

(11) **EP 4 210 067 A1**
(43) Date of publication of application: **12.07.2023**
(21) Application number: 21864350.0
(22) Date of filing: 01.09.2021
(51) Int. Cl.: G16H 20/70, G16H 50/20

(54) **MEDICAL TREATMENT SYSTEM AND METHOD FOR IMPLEMENTING SAME**

(30) Priority: 01.09.2020 JP 2020146925
(71) Applicant: WASEDA UNIVERSITY, Shinjuku-ku Tokyo 169-8050 (JP); Logos Science Corporation, Tokyo, 105-0001 (JP)
(72) Inventor: KUMANO Hiroaki, Tokyo 169-8050 (JP); SAITO Junichi, Tokyo 169-8050 (JP); URUSHIHATA Naoki, Tokyo 135-0064 (JP); TANEMURA Hideki, Tokyo 105-0001 (JP); GHAZIZADEH Mohammad, Tokyo 105-0001 (JP); SHIMOKAWA Chigusa, Tokyo 105-0001 (JP)
(74) Representative: Dehns
(86) International application number: PCT/JP2021/032058
(87) International publication number: WO 2022/050293

(57) **Abstract**

The present invention is a medical treatment system using an information processing terminal and an information processing network, and a method using said system, wherein the objective of the invention is to provide: a system that performs optimal medical treatment to a patient on the basis of a diagnostic cross-sectional assessment and/or a disease-specific assessment, such that treatment using process-based CBT is possible; and a method using said system. Toward this end, this system (100) has: a block (10A1: diagnostic cross-sectional assessment part) having a function for, while using normal lifestyle date, assessing the degree to which a patient is adaptively behaving, or non-adaptively behaving, in response to psychosocial issue, lifestyle adaptation, stress, etc.; and/or a block(10A2: disease-specific assessment part) having a function for assessing the degree to which the patient is adaptively behaving, or non-adaptively behaving, in response to the characteristic symptoms of a specified disease; and a block (10B:module selection part) having a function for analyzing the result of the assessment of blocks, classifying the patient into a specific group (stack or group), and selecting a medical treatment module linked with the classified group.

## Description

### TECHNICAL FIELD

The present invention relates to a medical technology for diagnosis, medical treatment (therapy), prevention, recurrence prevention, early detection, detection of complications of diseases, and the like through an information processing terminal (e.g., smart phone) and an information processing network system (e.g., Internet), without a direct meeting between a doctor and a patient.

### BACKGROUND ART

In recent years, there are developed technologies (so-called "medical treatment applications" or "digital therapeutics") for treating through information processing terminals and information processing network systems, without a direct meeting between a doctor and a patient (e.g., Patent Literature 1 and Patent Literature 2).

Such technologies make it possible to treat patients living in depopulated or remote areas where it is difficult to access to medical facilities. Moreover, it is possible to improve a consultation rate even for patients do not want to be medically treated by a specialized physician or a specialist because they want to keep a fact that "they are being treated" as confidential as possible due to privacy concerns.

Systems according to the prior arts (e.g., Patent Literatures 1 and 2) are constructed so as to identify disease and to focus on the presence or absence characteristic symptoms of the disease and it is a basic stance for providing the identical medical treatment for all patients, but there is a high possibility that medical treatment will be a deadlock for patients who are not treated successfully.

Cognitive Behavioral Therapy (CBT) is highly versatile for physical disorder and mental disorder, and therapeutic effects thereof are attracted much attention. For the CBT, in particular process-based Cognitive Behavioral Therapy (process-based CBT), it is important to understand patients on the basis of factors common to various diseases, such as psychosocial issues, lifestyle adaptation, and stress, instead of focusing merely on the presence or absence of characteristic symptoms of the disease. For this end, it is essential to assess (evaluate) a degree to which the patient is adaptively behaving or non-adaptively behaving in response to psychosocial issues, lifestyle adaptation, stress, and the like, while utilizing normal lifestyle data (normal lifestyle adaptation), and to further assess a degree to which the patient is adaptively behaving or non-adaptively behaving also in response to characteristic symptoms of the disease. It would be possible to implement the optimal medical treatment to the patient on the basis of the individual assessment as described above, but such an assessment is not performed in the prior arts (e.g., Patent Literatures 1 and 2).

### CITATION LIST

### Patent Literatures

Patent Literature 1: Japanese Patent No. 6339298
Patent Literature 2: Japanese Patent No. 6245781

### DISCLOSURE OF INVENTION

### PROBLEM TO BE SOLVED BY THE INVENTION

The present invention was proposed in consideration of the above-mentioned problems of the prior arts. The objects of the present invention are to provide a medical treatment system for performing diagnosis, medical treatment, prevention, and the like by using an information processing terminal and an information processing network system, and a method using such a system, enabling provision of a medical treatment using process-based CBT in the network. More specifically, the object of the present invention is to provide a system for providing the medical care (implementing treatment) optimal for a patient and a method of implementing such a system, on the basis of a result of a diagnostic cross-sectional assessment for, while using normal lifestyle data, assessing a degree in which the patient is adaptively behaving or non-adaptively behaving, in response to psychosocial issues, lifestyle adaptation, stress, and the like, and/or a disease-specific assessment for assessing a degree in which the patient is adaptively behaving or non-adaptively behaving, in response to the characteristic symptoms of the disease.

### Solution to Problem

A system (100) according to the present invention is characterized comprising;
a server (10) operates (being constructed to function) as a control unit (1), and
an information processing terminal (3) used for a patient, wherein
the server (10) comprises:
   a block (10A1: the diagnostic cross-sectional assessment part) having a function for, by using normal lifestyle data , assessing a degree to which a patient is adaptively behaving or non-adaptively behaving (a degree how a patient is adaptively behaving or non-adaptively behaving), in response to (against to) psychosocial issues, lifestyle adaptation, stress, and the like, and/or, a block (10A2: disease-specific assessment part) having a function for assessing the degree to which the patient is adaptively behaving or non-adaptively behaving (a degree how the patient is adaptively behaving or non-adaptively behaving), in response to (against to) the characteristic symptoms of the disease; and
   a block (10B: module selection part) having a function for analyzing a result (results) of the assessment(s) (the assessment of the diagnostic cross-sectional assessment part 10A1 and/or the assessment of the disease-specific assessment part 10A2) of the block(s) (the diagnostic cross-sectional assessment part 10A1 and/or the disease-specific assessment part 10A2), for classifying the patient into a specific group (stack or group), and for selecting a medical treatment module corresponding to (e.g., linked to) the classified group.

In the system according to the present invention, it is preferably to construct a platform comprising both the diagnostic cross-sectional portion and the disease-specific portion.

Alternatively, a system (100) according to the present invention is characterized that comprising a server (10) operates (constructed to function) as a control unit (1), and an information processing terminal (3) used for a patient, wherein the server (10) comprises:
a block (10A1: diagnostic cross-sectional assessment part) having a function for assessing a degree to which (how) a patient is adaptively behaving or non-adaptively behaving, in response to (against to) psychosocial issues, lifestyle adaptation, stress, and the like, by using normal lifestyle data, and/or, a block (10A2: disease-specific assessment part) having a function for assessing the degree to which (how) the patient is adaptively behaving, or non-adaptively behaving, in response to (against to) the characteristic symptoms of the disease; or
a block (10B: module selection part) having a function for analyzing a result (results) of the assessment(s) (the assessments of the diagnostic cross-sectional assessment part 10A1 and/or the assessment of the disease-specific assessment part 10A2) of the block (the diagnostic cross-sectional assessment part 10A1 and/or the disease-specific assessment part 10A2), for classifying the patient into a specific group (stack or group), and for selecting a medical treatment module corresponding to (linked to) the classified group.

Here, the above-mentioned server (10) is constructed by a computer or other information processing machine.

The normal lifestyle data includes: psychological data collected by asking various questions in normal life; and physiological data and behavioral data, such as a heart rate, blood pressure, blood oxygen level, the number of steps, sleep time and sleep quality (deep sleep, shallow sleep and the like), active quantity (active mass), a behavior range (behavior history: e.g., measured and obtained by GPS), physiological data such as blood glucose levels, and behavioral data. That is, the normal lifestyle data includes psychology data, behavioral data and physiological data.

Moreover, the system (100) according to the present invention may comprise a device for obtaining normal lifestyle data (e.g., electronic device including an information processing function and a communication function, such as a smart phone and a personal computer (PC)).

In addition, the system can also comprise an information processing terminal (4) used in a medical facility side.

In the present invention, it is also possible to visualize a degree of "psychological flexibility" and "mindfulness" in Acceptance and Commitment Therapy (ACT) by utilizing the normal lifestyle data.

A method according to the present invention is characterized comprising the steps for, in a method for carrying out the above-described system (the system according to any one of the claims 1-3);
assessing a degree to which (a degree how) a patient is adaptively behaving or non-adaptively behaving in response to (against to) psychosocial issues, lifestyle adaptation, stress, and the like, by utilizing normal lifestyle data (S4); and
analyzing a result (results) of the assessment(s) (assessment of the diagnostic cross-sectional assessment part 10A1) of the block, classifying the patient into a specific group (stack or group), and selecting a medical treatment module linked to the classified group (S7 to S9).

In addition to the above, the method according to the present invention comprises a step for assessing a degree to which (a degree how) the patient is adaptively behaving or non-adaptively behaving, in response to (against to) the characteristic symptoms of the disease (S6: assessment of the disease-specific assessment part 10A2), wherein

in the step for selecting the medical treatment module (S7 to S9), it is also preferable to analyze a result of assessing the degree to which (a degree how) the patient is adaptively behaving or non-adaptively behaving, in response to (against to) the characteristic symptoms of the disease, and then, to classify the patient into the specific group (stack or group).

It is preferable for the present invention to apply issues of relationship with a spouse or a partner, erectile dysfunction, depression, obsessive-compulsive disorder, panic disorder, agoraphobia, generalized anxiety disorder, social anxiety and speech anxiety, specific phobic disorder (specific phobia), post-traumatic stress disorder, complex anxiety disorder, insomnia, irritable bowel syndrome, eating disorder and obesity, obesity and other lifestyle-related diseases (lifestyle disease), bipolar disorder, borderline personality disorder, attention-deficit hyperactivity disorder, chronicity or persistent pain (ache), sexual dysfunction, issues of relationship with a spouse or a partner except for (without) erectile dysfunction, alcoholism and other addictions, schizophrenia and other severe psychoses, generalized anxiety, social anxiety, speech anxiety and other anxiety, specific phobic disorder (specific phobia), infertility (male, female), atopic dermatitis, overactive bladder (urinary incontinence), anti-aging, menopausal disorder, premenstrual syndrome (PMS), psychogenic visual disturbance, dental psychosomatic disease, dysmorphic phobia (body dysmorphic disorder), and the like.

Upon carrying out the present invention, in the case of psychogenic ED, it is possible to utilize indices, for example three indices "Psychological Flexibility in ED" AFQ-ED, "Sexual Communication with Partner" SRQ, and "General Psychological Flexibility" PFQ, for the classification of the specific group (cluster analysis).

In the present invention, it is preferable to be constructed so that patient's medical treatments and behaviour such as medication are recorded, and that a medical doctor can describe the records (e.g., the records of patient's medication (medical treatment)) and the behavioral records (e.g., record regarding sexual activity in the case of ED patient) on each date of a check calendar, and it is preferable to be constructed so that the medical doctor can check the records on each date of the calendar.

Moreover, a medical examination (including an outpatient medical examination and an online medical examination) by the medical doctor can be carried out every predetermined period (e.g., two weeks) to determine a treatment policy, prescription of drugs used for the medical treatment, and the like. At the time of the medical examination, the assessment (diagnostic cross-sectional assessment, disease-specific assessment) can be carried out, the classification of the patient into the specific group based on the cluster analysis can be carried out, and work and the like to be provided to the patient can be newly determined. A result of the assessment(s) which is (are) carried out again can also be utilized in medical examinations performed by the medical doctor. Here, the medical examinations performed by the medical doctor, the newly determined medical treatment (therapy) policy, the drug prescription and the like are recorded.

It is possible to change the treatment policy, prescription, work content and the like, in response to changes in the normal lifestyle data and EMA, without changing the specific group (e.g., cluster 1) in which the patient belongs.

By providing new work, it is possible for each patient to provide the optimal medical treatment.

### EFFECT OF THE INVENTION

In accordance with the present invention comprising with the above-described constructions, by the diagnostic cross-sectional assessment, it is possible to assess the degree to which (the degree how) the patient is adaptively behaving or non-adaptively behaving against to psychosocial issues, lifestyle adaptation, stress, and the like, while utilizing normal lifestyle data.

Furthermore, by the disease-specific assessment, it is possible to assess the degree to which (the degree how) the patient is adaptively behaving or non-adaptively behaving against to characteristic symptoms of the disease. Based on these assessments, it is possible to provide the optimal medical treatment module for the patient. In particular, it is possible to provide extremely effective medical care (medical treatment, diagnosis, prevention / recurrence prevention / early detection / detection of complications of diseases, and the like) for cases which are not improved by identifying and treating the disease merely.

Here, in the present invention, "process" is focused rather than "result". For example, in the case of a patient with psychogenic ED, it is focused whether the psychological "process" stage of the patient has progressed and thereby the patient's use of the mind has improved, rather than whether the symptom of "erectile dysfunction" (corresponding to the "result" in this case) has improved. If the stage of the psychological process progresses and the patient's way of thinking improves, it means that a new behavioral pattern has been acquired and learned. Accordingly, in the case of the above-mentioned patient whose behavioral pattern has been acquired and learned, a range of the way of thinking said patient can be widen, such as "it's okay if cannot get an erection" and "the relationship with my partner is not all about sexual activity". As a result, the psychogenic ED is improved or cured, and the quality of life is improved.

In accordance with the present invention, it is possible to realize a certain effect by providing the optimal medical treatment module, and thereby, it is possible to execute the medical treatment with the process-based CBT, and then, it is possible to customize the medical treatment for every patient or every type of the diseases. By carrying out the diagnostic cross-sectional assessment, the disease-specific assessment and the cluster analysis with quantitative measurement indices, the medical treatment is optimized. For example, it is possible to exert therapeutic effects (cure effects) for issues of relationship with a spouse or a partner, erectile dysfunction, depression, obsessive-compulsive disorder, panic disorder, agoraphobia, generalized anxiety, social anxiety and speech anxiety, specific phobic disorder, post-traumatic stress disorder, insomnia, irritable bowel syndrome, eating disorder and obesity, obesity and other lifestyle-related diseases, bipolar disorder, borderline personality disorder, attention-deficit hyperactivity disorder, chronicity or persistent pain, sexual dysfunction, issues of relationship with a spouse or a partner without erectile dysfunction, alcoholism and other addictions, schizophrenia and other severe psychoses, infertility (male, female), atopic dermatitis, an overactive bladder (urinary incontinence), anti-aging, menopausal disorder, premenstrual syndrome (PMS), psychogenic visual disturbance, dental psychosomatic disease, body dysmorphic disorder (dysmorphic phobia), and the like. It is also effective for prevention of patient suicide. Furthermore, the therapeutic effect can also be exerted in a case that complications are generated from the above-described diseases.

Moreover, in accordance with the present invention, it is also possible to carry out an assessment (assessments) in a case that there are a plurality of complications of disease symptoms/conditions, and to propose or provide the optimum diagnostic cross-sectional treatment method. For example, in an application for psychogenic ED, it is possible to analyze a tendency of complication, such as depression, anxiety, and diabetes, from a result of the assessment. Alternatively, assessment for menopausal disorder can be exerted based on a plurality of indefinite complaints.

Furthermore, if the patient can behave adaptively to the psychosocial issues, lifestyle adaptation, stress and the like by applying the present invention, the present invention can be also applied to prevention of infection such as coronavirus, after-coronavirus anxiety and the like.

Moreover, a higher therapeutic effect can be expected by combining the medical treatment application (digital therapeutics) according to the present invention with a regenerative medicine using stem cells or a medical treatment using a supernatant of the stem cells.

In addition, in accordance with the present invention, since the quantitative measurement indices, the objective monitoring methods, and the cluster analysis can be used, it is possible to clearly explain an algorithm of the program to a third party, and thereby, it is easy to facilitate the use of actual results as evidence. Therefore, it is easy to prepare necessary materials (objective evidentiary data, description of a system used therefor, and the like) to be submitted for various kinds of approval.

The system or method according to the present invention can be used by multiple people together. Then, a partner of a patient of ED or infertility (male or female), a family member, a helper of a patient of depression or anxiety disorder or the like can share information and contents, which are required for medical treatment and the like with the patient, and can execute exercises related to the medical treatment module in collaboration with the patient. Thereby, the partner, the family member, the helper or the like can accompany with the patient, and can deepen their understanding and practice of the disease and how to help.

Then, the patient can participate actively in the determination of the treatment policy and can receive medical treatment in accordance with the determination, in which situation "adherence" can be established, and thereby, the therapeutic effect can be improved.

In accordance with the present invention, operative effects of which are above-mentioned, it is possible to personalize the medical treatment.

### BRIEF DESCRIPTION OF DRAWINGS

[Fig. 1] Fig. 1 is a functional block diagram of an overview of a system according to an embodiment of the present invention.
[Fig. 2] Fig. 2 is a functional block diagram of a server which is used in the embodiment.
[Fig. 3] Fig. 3 is a functional block diagram of a module selection part shown in Fig. 2.
[Fig. 4] Fig. 4 is a functional block diagram of a patient-side electronic device which is used in the embodiment.
[Fig. 5] Fig. 5 is a functional block diagram of a medical institution (medical facility) side electronic device which is used in the embodiment.
[Fig. 6] Fig. 6 is a flow chart which shows procedures in a first access of patient of in the control of the embodiment.
[Fig. 7] Fig. 7 is a flow chart which shows procedures in a second and subsequent accesses of patient in the control of the embodiment.

### BEST MODE(S) FOR CARRYING OUT THE INVENTION (EMBODIMENT)

Embodiments of the present invention will be explained in reference with the attached drawings, in followings. The illustrated embodiment describes a case where a process-based CBT is applied to medical care (medical treatment (therapy), diagnosis, prevention, recurrence prevention, early detection, detection of complications of diseases, and the like) of patients with psychogenic erectile dysfunction (ED). However, the present invention can be applied not only to psychogenic ED but also to other sexual dysfunctions, issues of relationship with a spouse or a partner, depression, obsessive-compulsive disorder, panic disorder, agoraphobia, generalized anxiety disorder, social anxiety and speech anxiety, specific phobic disorder (specific phobia), post-traumatic stress disorder, insomnia, irritable bowel syndrome, eating disorder and obesity, obesity and other lifestyle-related diseases (lifestyle diseases), bipolar disorder, borderline personality disorder, attention-deficit/hyperactivity disorder, chronicity or persistent pain (ache), sexual dysfunction, issues of relationship with a spouse or a partner except for erectile dysfunction, alcoholism and other addictions (dependence), schizophrenia and other severe psychoses, infertility (male, female), atopic dermatitis, an overactive bladder (urinary incontinence), anti-aging, menopausal disorder (menopause), premenstrual syndrome (PMS), psychogenic visual disturbance, dental psychosomatic disease, body dysmorphic disorder (dysmorphic phobia), and the like. Moreover, the present invention can be applied to complications arising from the above-described diseases.

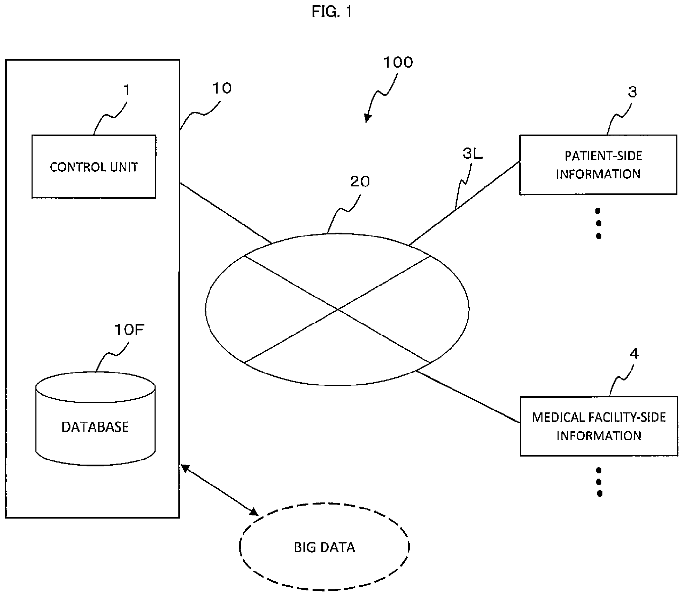

In Fig. 1, a system entirely shown by the reference number 100 comprises a server 10 (e.g., a computer) including a control unit 1 and a database 10F, an information processing terminal 3 being used for a patient (patient-side information processing terminal), and an information processing terminal 4 being used in a medical facility (medical facility-side information processing terminal), in which system these components are interconnected, for example, by an information processing network 20 (e.g., a local area network or the Internet).

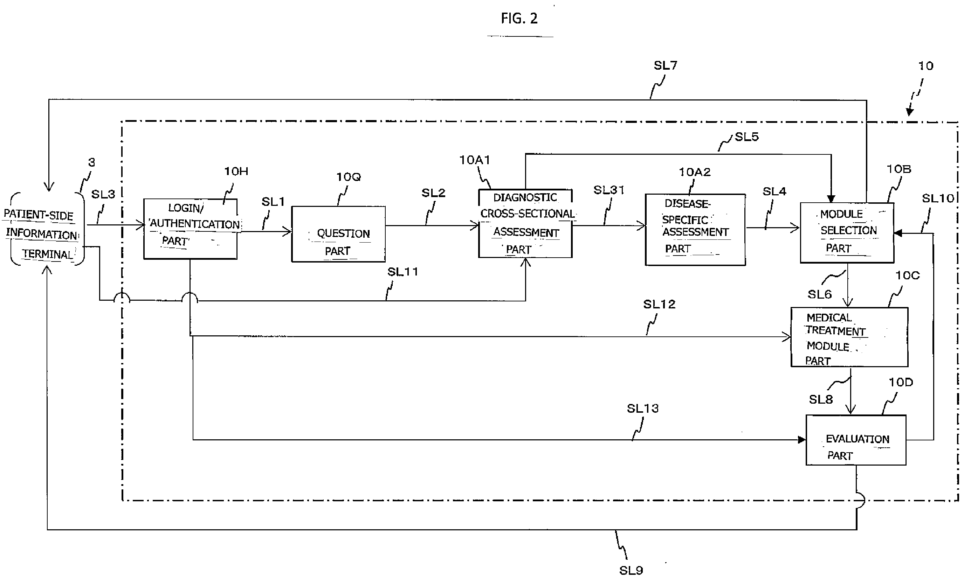

The details of the server 10 will be described below with reference to Fig. 2, and the control unit 1 shown in Fig. 1 corresponds to a functional block diagram which illustrates various parts comprehensively in Fig. 2.

Here, the term "patient" used in this specification means a person who individually recognizes oneself that he/she has a possibility of having suffered from psychogenic ED or the like, and has an intention of being treated. In other words, the term "patient" is not limited only persons who are undergoing the medical treatment by guidance of a medical doctor and another relevant person in a medical facility, but may also include persons who would be determined to be "healthy" by a third party.

In Fig. 1, a patient-side information processing terminal 3 is constructed with a "device for obtaining normal lifestyle data", e.g., an electronic device having an information processing function and a communication function, such as a smart phone or a personal computer (PC). Alternatively, wearable device or the like can also be used as a device for obtaining the normal lifestyle data. The normal lifestyle data includes psychological data which can be collected by asking various questions in normal life. Moreover, the normal lifestyle data comprises physiological data, such as a heart rate, blood pressure, blood oxygen level, number of steps, sleep time and sleep quality (deep sleep, shallow sleep, and the like), active mass, a behavior range (behavior history or action history: e.g., measured and obtained by GPS), and physiological data such as blood glucose levels, and behavioral data.

As the medical facility-side information processing terminal 4, an electronic device (e.g., PC) having an information processing function and a communication function can be used, and also, a smart phone can be used.

Here, the patient-side information processing terminal 3 (e.g., a smart phone) is not only used as a tool for accessing the server 10 through a line 3L and the network 20, but also has functions for a tool for measuring a patient's "normal lifestyle data " to be transmitted to the server 10 side in order to be utilize for the diagnostic cross-sectional assessment in the server 10. The function for measuring the normal lifestyle data and transmitting the data to the diagnostic cross-sectional assessment part in the server 10 can be carried out by installing a dedicated application for executing such a function on a smart phone, which is the patient-side information processing terminal 3.

When such the dedicated application is installed, the information transmission line 3L (which may be wired or wireless) for connecting the patient-side information processing terminal 3 with the network 20 is both an access line for accessing the server 10 and an information transmission line for transmitting the patient's normal lifestyle data measured by the information processing terminal 3 to the server 10.

The database 10F in the server 10 stores patient information (e.g., medical practice history information corresponding to a patient's ID) and information regarding psychogenic ED medical treatment (treatment methods, information regarding a PDE5 inhibitor including a medication history, and information regarding the process-based CBT, and the like). It is to be noted that the medical practice in the medical practice history information includes a medical practice via information terminals and a medical practice at medical facilities.

Although not shown in Fig. 1, a plurality of patient-side information processing terminals 3 and a plurality of medical facility-side information processing terminals 4 can also be connected to the network 10.

In Fig. 1, the database 10F is built into the server 10 together with the control unit 1. However, the database 10F can also be constructed separately from the server 10 and can be connected to the server 10 through the network 20.

The server 10 can be coupled to a big data or a system 30 using the big data. By being coupled to the big data or the system 30 using big data, results of selection of assessment and medical treatment module in the server 10, a situation of medical treatment, and the like can be utilized as a part of the big data, and it becomes also possible to make use of knowledge obtained by utilizing the big data in order to improve the server 10.

Although not shown, it is also possible to incorporate the knowledge and information obtained by utilizing the above-described big data into construction of Artificial Intelligence (AI) algorithm so as to develop medical applications (digital therapeutics) using AI.

The server 10 in the system 100 according to the illustrated embodiment will now be described with reference to Fig. 2, which shows a functional block.

In the illustrated embodiment, a diagnostic application (an assessment module) and a medical treatment / prevention application (a medical treatment module and an evaluation module) are shown as an integrated unit. However, the diagnostic application (the assessment module) and the medical treatment / prevention application (the medical treatment module and the evaluation module) can be constructed separately so that said applications are separated from each other, and that the medical treatment / prevention application (the medical treatment module and the evaluation module) prescribes (including a case in which a medical doctor prescribes) with respect to the assessment result of the diagnostic application (the assessment module).

In Fig. 2, the server 10 (shown by the dashed line in Fig. 2) includes an assessment part 10A, a module selection part 10B, a medical treatment module part 10C, an evaluation part 10D, and a login/authentication part 10H. Although not clearly shown, in the illustrated embodiment, a platform is constructed which comprises both a diagnostic cross-sectional assessment part 10A1 and a disease-specific assessment part 10A2.

Moreover, in Fig. 2, the information transmission lines SL1 ... may be wireless as well as wired.

The login/authentication part 10H corresponding to an entrance of communication from the patient-side terminal 3 to the server 10 carry out of an authentication of the patient when an ID and a password are entered from the patient-side information processing terminal 3. The login/authentication part 10H confirms validity and reliability of the patient on the basis of previous data, and transmits an authentication result (information indicating that the patient is a valid patient, not a fake) to a question part 10Q.

Although not clearly shown, in the server 10, security measures, such as a preventing impersonation (spoofing) of patients, are carried out by using known art for example. In the illustrated embodiment, such security measures are applied to the login/authentication part 10H to protect the system 100 from impersonation of patients and other fraudulent activities. In a case that a person who access to the system 100 is an impersonator pretending to be a patient, the access thereof is blocked in the login/authentication part 10H, and information transmission to the question part 10A and subsequent parts are blocked.

The login/authentication part 10H, for example, has a function for issuing a prescription ID and a password and confirming the identity of the accessor (patient). Although not shown, it is possible, for example, with the patient's consent, to appropriately set the threshold levels of times of accessing the system 100 and the period of time since the first access, and to deactivate or restrict the access if the number of times or the set period of time is exceeded the threshold level. As will be described later, the system can be constructed so that, even if being deactivated, the patient can access the system 100 again in a case that there is a doctor's instruction.

Although not shown, at the time of login authentication, in addition to input of the ID and the password, if the patient-side information processing terminal 3 is a smart phone or a PC, a biometric information unique to the patient, for example, such as the patient's face, voice (voiceprint), retina, and iris, can be obtained by means of a camera of said smart phone or PC, and authentication of the patient can be performed using the biological information of the patient. As a result, so-called "impersonation (spoofing)" and account transfer can be prevented and thereby the reliability of data input or obtained from the patient can be ensured. It is possible to obtain the biological information as described above not only at the time of login but also at the time of inputting (obtaining) patient's data.

The question part 10Q connected to the login/authentication part 10H through the information transmission line SL1 makes the accessing patient (subject) to input a chief complaint, a current medical history, a past medical history, and the like. Simultaneously, the question part 10Q interviews the accessing patient on the basis of evaluation of psychogenic ED (e.g., "When starting thinking that I'm going to fail again, that's all I can think about.", "I can't concentrate on sexual activity because I worry about whether I'll be able to maintain an erection.") and criteria (operational criteria: operational reference) for classifying groups of psychological dysfunctions (psychiatric symptoms). Specifically, the question part 10Q carries out asking questions for the accessing patient in the form of, for example, a symptom checklist for psychogenic ED and a checklist based on DSM-5 and ICD-10 compliant psychiatric simplified structured interview method.

On the basis of the input and the response in the accessing patient's question part 10Q, it is possible to determine whether or not a specified disease is manifested and to determine the presence or absence of a risk-factors, which can be useful for the assessment in the assessment parts 10A1 and 10A2.

The input and the response in the question part 10Q are transmitted to the diagnostic cross-sectional assessment part 10A1 through the information transmission line SL2.

The diagnostic cross-sectional assessment part 10A1 has a function for assessing a degree to how the patient is able to behave adaptively or non-adaptive, in response to psychological social problems, lifestyle, and stress, and the like, by utilizing the normal lifestyle data obtained by the patient-side information processing terminal 3 (device for obtaining the normal lifestyle data), and the assessment part 10A1 carry out assessment by using the quantitative measurement reference (indices). For example, the following adaptively behaviors are assumed:
"Acceptance", which is a behaviour of not avoiding unpleasant thinking and feeling and leaving them as it is;
"Defusion", which is a behaviour of distinguishing between the thinking and the reality and of not being engulfed by unpleasant thinking or feeling;
"Contact with the Present Moment", which is a behaviour of holding an awareness of reality in front of us;
"Self as Perspective (Self of Visual Point)", which is a behaviour of noticing what oneself is noticing from a bird's-eye viewpoint;
"Values Clarification", which is a behaviour of clarifying the "way of life" which wants to live;
"Commitment", which is a behaviour of pursuing the "way of life" which wants to live; and the like.

The psychological flexibility of the accessing user (patient: subject) is assessed by assessing the above-described adaptively behaviour in response to psychosocial issues, lifestyle adaptation, stress, and the like. The assessments for the above-described six adaptive behavior are carried out based on an Acceptance and Commitment Therapy (ACT), which is one of the CBT. Then, if a subject who behave the above-described adaptively behavior in response to the psychosocial issues, lifestyle adaptation, stress, and the like, it can be determined that said subject is healthy and has a high quality of life, low stress, and high resistance to various diseases.

The assessment result obtained in the diagnostic cross-sectional assessment part 10A1 is transmitted to the module selection part 10B through the information transmission line SL5.

The diagnostic cross-sectional assessment part 10A1 does not assess identification of a specific disease, nor does not assess characteristic symptoms of the disease. As described above, the diagnostic cross-sectional assessment part 10A1 assesses the degree to how the patient is able to behave adaptively or non-adaptive, in response to psychosocial issues, lifestyle adaptation, stress, and the like, by utilizing normal lifestyle data. As a result of carrying out such the assessment, in the diagnostic cross-sectional assessment part 10A1, it is possible to understand the patient on the basis of factors (parameter) which is common to various diseases, such as psychosocial issues, lifestyle adaptation, and stress, and therefore, the process-based CBT can be executed.

It is one of the important features of the illustrated embodiment to understand the patient in the diagnostic cross-sectional assessment part 10A1 on the basis of the factors which is common to various diseases, such as psychosocial issues, lifestyle adaptation, and stress, and said feature is clearly difference point from various digital therapeutics (medical treatment application) of prior art (e.g., refer to Patent Literatures 1 and 2).

The information of the subject, which information is obtained after the assessment of the diagnostic cross-sectional assessment part 10A1 is completed, is transmitted to a disease-specific assessment part 10A2 through information transmission line SL31.

The disease-specific assessment part 10A2 has a function for assessing a degree to how the subject (accessing user) behaves adaptively or non-adaptively, in response to the characteristic symptoms of the disease thereof, and the part 10A2 carries out the assessment by using the quantitative measurement indices. For example, the following non-adaptively behaviors are assumed:
"Anticipatory Anxiety", which is a behavior of beginning to think that sexual activity may fail;
"Self-Concept", which is a behavior of feeling to be disqualified as a "man" if erection is unmaintainable during sexual activity;
"Attention Control (Self-Regulation)", which is a behavior of being not able to concentrate on sexual activity since the subject worries about whether I'll be able to maintain an erection;
"Interpersonal Relationships", which is a behavior of being concerned about evaluation from partner and not establishing a favorable relationship;
"Avoidance Behavior", which is a behavior of being not able to attempt a sexual activity; and the like.
Moreover, in a case of depression, the following non-adaptively behaviors are assumed:
   "Inactivity (Inertness)", which is a state of inactivity and reduced range of activity;
   "Rumination", which is a behavior of thinking over and over about past unpleasant events;
   "Cognitive Bias (Cognitive Distortion)", which is a behavior such as thinking things as the extreme situation and denying everything based on the slightest mistake;
   "Social Dysfunction (Impairment of Social Functioning)", which is behavior of avoiding from interacting with others; and the like.

In the disease-specific assessment part 10A2, in a case that the subject is assessed for the above-described non-adaptively behavior in response to the characteristic symptoms of the disease, the information relating to the subject is transmitted to the module selection part 10B together with the assessments of the assessment parts 10A1 and 10A2 through the information transmission line SL4.

Here, in a case that the subject has no signs of a specified disease (e.g., in a case that the subject is a healthy person) as a result of the intake information and the medical interview in the question part 10Q, a disease-specific assessment is not performed in the illustrated embodiment. In such a case, the information of the subject obtained by the diagnostic cross-sectional assessment part 10A1 is transmitted to the module selection part 10B together with the result of the assessment result of the diagnostic cross-sectional assessment part 10A1 through the information transmission line SL5, which line is bypassing the disease-specific assessment part 10A2. Moreover, in a case that that the subject is determined as a healthy person by a result of the intake information and the medical interview in the question part 10Q, it is possible to classify the subject into a cluster corresponding to a healthy person, without being assessed in the diagnostic cross-sectional assessment part 10A1.

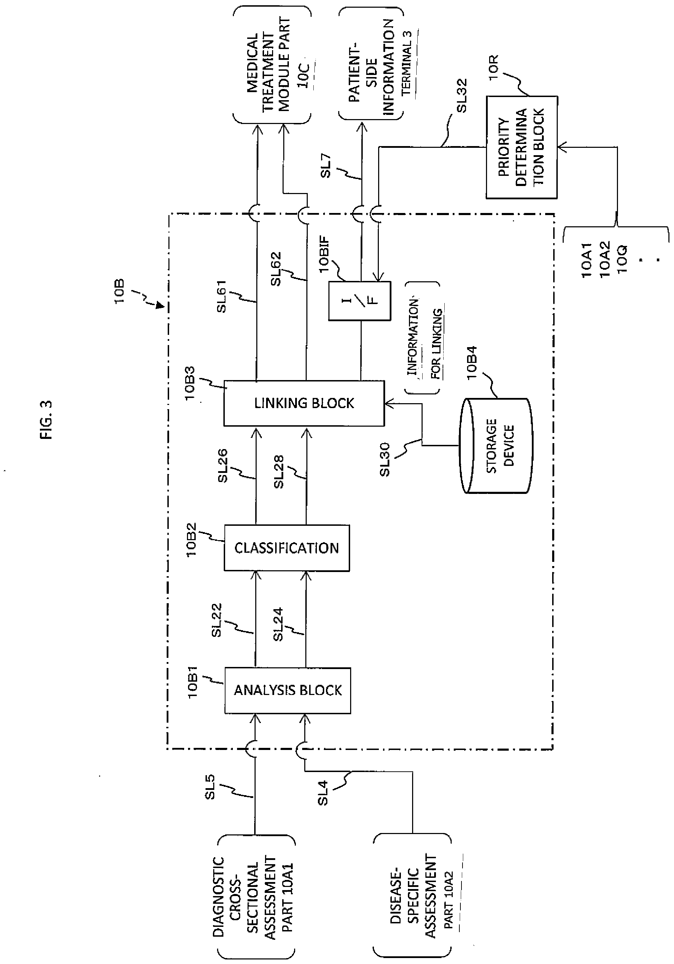

As described with reference to Fig. 3, the module selection part 10B classifies a subject into a specific group using statistical methods (e.g., cluster analysis) (the part 10B classifies the subject into a cluster in the case of cluster analysis), and selects medical treatment module linked to the concerning group or the concerning cluster. In other words, the module selection part 10B has a function for selecting the medical treatment module on the basis of a result of the assessment performed in the diagnostic cross-sectional assessment part 10A1 and/or a result of the assessment performed in the disease-specific assessment part 10A2. For example, in the assessment performed in the diagnostic cross-sectional assessment part 10A1, as clusters of the "Acceptance", the "Defusion", the "Contact with the Present Moment", "Self as Perspective", "Values Clarification", and the "Commitment", a patient is classified into the following four clusters:
cluster 1 (scores of the "Acceptance" and the "Defusion" are lower than an average level);
cluster 2 (scores of the "Contact with the Present Moment" and the "Self as Perspective" are lower than the average level) ;
cluster 3 (scores of the "Values Clarification" and the "Commitment" are lower than the average level); and
cluster 4 (score of the "Acceptance", the "Defusion", the "Contact with the Present Moment", and the "Self as Perspective" are lower than the average level).

The average level means the average score ±1 SD (standard deviation). The patient is classified into any one of the above-described clusters on the basis of a result of the assessment performed in the diagnostic cross-sectional assessment part 10A1.

For psychogenic ED, in the assessment performed in the disease-specific assessment part 10A2, as a cluster of the "Anticipatory Anxiety", the "Self-Concept", the "Attention Control", the "Interpersonal Relationships", and the "Avoidance Behavior", a patient is it classified into the following four clusters:
cluster 1 (scores of the "Anticipatory Anxiety" and the "Attention Control" are higher than the average level);
cluster 2 (scores of the "Anticipatory Anxiety" and the "Avoidance Behavior" are higher than the average level);
cluster 3 (scores of the "Self-Concept" and the "Interpersonal Relationships" are higher than the average level); and
cluster 4 (scores of the "Interpersonal Relationships" and the "Avoidance Behavior" are higher than the average level). The patient is classified into any one of the above-described clusters on the basis of a result of the assessment performed in the disease-specific assessment part 10A2.

Moreover, in the above-described example, the cluster analysis is carried out based on each assessment result of the diagnostic cross-sectional assessment part 10A1 and the disease-specific assessment part 10A2, but a result of the assessment performed in the diagnostic cross-sectional assessment part 10A1 and a result of the assessment performed in the disease-specific assessment part 10A2 may also be integrated with each other in order to carry out the cluster analysis.

Then, the module selection part 10B selects the medical treatment module on the basis of the cluster in which the patient is classified on the basis of the result of the assessment performed in the diagnostic cross-sectional assessment part 10A1 and the result of the assessment performed in the disease-specific assessment part 10A2.

In the above-described example, the number of the clusters is four, but the number of clusters is assumed to be 1000 or more due to customization of data analysis and the medical treatment applications, in an actual cluster analysis.

In the illustrated embodiment, since the platform is constructed to comprise each of the diagnostic cross-sectional assessment part 10A1 and the disease-specific assessment part 10A, even if the system 100 is constructed so that the diagnostic application and a medical treatment / prevention application are separated from each other, the medical treatment module based on the result of the diagnostic cross-sectional assessment and/or the medical treatment module based on the result of disease-specific assessment are automatically selected and instructed to each cluster which is determined depended on the cluster determination in the assessment part (assessment module) in the diagnostic application. Here, even in the system 100 constructed so that the diagnostic application and the medical treatment / prevention application are integrated with each other as a single unit or even in the case where they are separated from each other, the medical doctor can manually select and instruct the medical treatment module on the basis of the result of the assessment, for selection and instruction of the medical treatment module.

The medical treatment module selected in the module selection part 10B is transmitted to the medical treatment module part 10C through the information transmission line SL6.

The module selection part 10B has a function for transmitting the selected medical treatment module to the patient-side information processing terminal 3, through an interface which is not shown and the information transmission line SL7. That is, if the patient's cluster is determined and the medical treatment module is indicated as a result of assessment, a part of a medical treatment program (exercise, work, and the like) of the medical treatment module, homework, and information (Tips) useful to the medical treatment can be transmitted to the patient-side terminal 3. In accordance with such a function, said medical treatment program can be executed even at a location where information net environment is not prepared or off-line. Accordingly, for example, even if a problem occurs in communication/network environment or information communication is impossible (including a case that the communication is non-activated) for accessing a system 100 after medical treatment is completed, the patient can continue to execute and browse successively through off-line a part of exercise and homework, and information (Tips) useful to the medical treatment, which are performed in the medical treatment module, thereby, an interruption of the medical treatment is prevented and the patient is promoted to continue the therapeutic effect and to prevent a recurrence after the medical treatment is completed.

However, the exercise, the workout, the homework, and the Tips performed off-line are not recorded in the system 100 side as a record of intervention. Accordingly, in order to prevent a possibility that appropriate evaluation is impossible, although being not shown, it is possible to be constructed to have a function for transmitting the exercise, the workout, the homework, and the Tips which are performed in off-line to the system 100 side.

Here, in a case that a patient gets psychogenic ED, for example, there is a work using a known Vacuum Constriction Device (VCD) as one example of the above-described work. It is preferable to exercise the above-mentioned work with a partner (spouse or the like), and, the fact that the patient has an erection to the extent that a sexual intercourse is possible, and the fact that a sexual intercourse is successfully performed, produces a positive effect on recovery of the patient's confidence.

The medical treatment module part 10C has a function for making the patient to carry out the medical treatment module selected in the module selection part 10B, or for transferring the medical treatment module selected in the module selection part 10B to the patient.

The medical treatment module being executed in the medical treatment module part 10C provides psychological intervention for aiming learning/acquisition of a healthy and new behavioral pattern in response to a result of the assessment result of the "Psychological Flexibility" in the diagnostic cross-sectional intervention. For example, "Polar Bear Exercise (exercise relating to White Bear Principle)" or the like is executed to a patient who is implemented classified into the cluster 1 (scores of the "Acceptance" and the "Defusion" are lower than the average level).in the assessment performed in the diagnostic cross-sectional assessment part 10A1. The object of the "Polar Bear Exercise" is to understand the nonfunctionality of trying to avoid unpleasant thinking and feeling by trying not to think about the polar bear for a predetermined time. If a patient is classified into the cluster 2 (scores of the "Contact with the Present Moment" and the "Self as Perspective" are lower than the average level), "Attention Training ("Attention Training Technique)" or the like is carried out. The object of the "Attention Training" is to obtain attentional flexibility through a practice of directing attention to a plurality of sounds by various methods and to release oneself from self-attentive (self-focus) cognitive processing. By carrying out these medical treatment modules, it can be expected that the adaptively behavior included in the cluster is promoted. It is to be noted that, in this specification, the implementation of the psychotherapy may be written as an intervention for the patient.

Moreover, as a medical treatment module for psychogenic ED, the disease-specific intervention provides psychological intervention for aiming learning/acquisition of healthy and new behavioral pattern according to the concerning disease by using appropriate technique for each cluster, in response to a scene, a situation, etc. peculiar to each disease. For example, in the assessment performed in the disease-specific assessment part 10A2, if a patient is classified into the cluster 1 (scores of the "Anticipatory Anxiety" and the "Attention Control" are higher than the average level), "Sensate Focus Training (Sense Concentration Exercise)" is carried out. In the "Sensate Focus Training", a patient contacts to the partner's body, and an object thereof is focusing every nerve on a given sensation. If a patient is classified into the cluster 3 (scores of the "Self-Concept" and the "Interpersonal Relationships" are higher than the average level), "Partner Relationships (Relationships whit Partner)" is carried out. The object of the "Partner Relationships" is to find an area in which it is possible to discuss and compromise being important for maintaining a sexual relationship.

In the medical treatment module part 10C, it is possible to provide a medical treatment module using "Virtual Reality". For example, in the case of an anxiety disorder patient, it is possible to make the patient experience (virtual reality experience) "get aboard a train" through virtual reality.

In the illustrated embodiment, the diagnostic cross-sectional intervention is prior to the disease-specific intervention. However, there are cases in which the above-mentioned relationship is not applied.

A homework to be carried out by the patient is prepared in the medical treatment module part 10C, corresponding to the cluster to which the patient is classified. The homework to be selected by the medical treatment module part 10C is stored in a storage device (database) not shown in Fig. 2, and it is possible to refer the homework linked to each medical treatment module. Such a homework is edited predetermined contents which can be expected to improve the symptoms when being carried out by the patient and patient's independence can be improved. For example, in the above-described medical treatment module "attention training", a patient exercises to pay attention to various sounds recorded in a sound source for approximately 15 minutes a day.

The medical treatment module and a medical treatment result being executed in the medical treatment module part 10C are transmitted to the evaluation part 10D through the information transmission line SL8. It is possible that there is a case that a homework and accomplishment status thereof are transmitted to the evaluation part 10D.

The evaluation part 10D has a function for evaluating an effect of the medical treatment produced by the medical treatment module being executed in the medical treatment module part 10C, and the part 10D evaluates the effect of the medical treatment in accordance with a predetermined algorithm by using a known evaluation scale (assessment scale) or the like. For example, in the psychogenic ED, the International Index of Erectile Function (IIEF) is used as an evaluation scale (measurement index or assessment index) for erectile function, which is a characteristic symptom of the disease. In the international index of erectile function, severity classification is carried out by scale scores, and it is possible to evaluate an effect of medical treatment. The evaluation scale or the measurement index as a tool is stored in the medical treatment module part 10D. Alternatively, the evaluation scale or the measurement index for each of a medical treatment module may be stored in a storage device (database) which is not shown in Fig. 2.

Here, it is possible to obtain and record patient's normal lifestyle data by using the patient-side terminal 3, a wearable device, or the like, and to make a result of effect measurements before and after the medical treatment into a report format. Moreover, it is possible for the evaluation part 10D to be able to have a function for carrying out such feedback to the patient (or medical doctor).

The evaluation result of the therapeutic effect by the evaluation part 10D is transmitted to the patient-side information terminal 3 through the information transmission line SL9. For example, in a case that a chatbot is used as the medical treatment module, evaluation of the therapeutic effect by the chatbot is displayed to the patient.

The evaluation contents transmitted to the patient includes, for example, advices for improving symptoms, and the like.

Although not shown in the drawings, when inputting responses of the measurement index of the medical treatment effect, it is preferable to be provided with a timing means and a recording means for measuring time period until the inputting; a measurement means for measuring a heart rate and a blood pressure; a means for measuring a state of patient's pupil opening and closing, a direction of a line of sight, and the number of times of blinks (e.g., a camera if the patient-side terminal 3 is a smart phone or PC); a measurement means for measuring the number of times of breaths; a measurement means for measuring body temperature; or a means for observing a complexion (e.g., camera of the patient-side terminal 3). Furthermore, it is preferable to be provided with a device for monitoring an objective information at the time of inputting the index, and measuring and observing accuracy of the response and a respondent's state.

By providing the device for measuring and observing the accuracy of the response and the respondent's state, the accuracy of the response and the respondent's state can be monitored objectively. The information as the respondent's states to be monitored are whether the patient, respondent has inputted the measurement index in haphazardly (carelessly); whether the patient is falsely inputting to obtain an "improved" result; whether the patient is stressed due to inputting the measurement index; whether the patient is deep in thought; patient's responsiveness; patient's cognitive function; and the like, for example. The data obtained from such monitoring can be incorporated as a factor utilizing in the cluster analysis.

Moreover, in a case that a camera of a smart phone or PC as the patient-side terminal 3 is used as the device for measuring and observing the accuracy of the response and the respondent's state, the above-mentioned monitoring can be carried out by attaching a dedicated filter, a specific lens, and the like to the camera. Furthermore, it is possible to use a measuring device (not shown) and application for measuring a heart rate, a blood pressure, and the like.

Furthermore, it is possible to obtain respondent's objective information by wearing devices, such as a smell sensor (odor sensor), an electroencephalogram sensor, and a cerebral blood flow sensor, and the like to a patient. Thereby, it is also possible to measure the respondent's physical condition to be incorporated as a factor or parameter in the cluster analysis. For example, if wearing a headset-like device for measuring brain waves, cerebral blood flow, and the like to a patient, the real feeling of the medical treatment is increased and the therapeutic effect is further improved.

In the evaluation part 10D, in a case that a favorable evaluation is not obtained with regard to the therapeutic effect, such the evaluation result is transmitted to the module selection part 10B through the information transmission line SL10. In a case that an unfavorable evaluation is received from the evaluation part 10D, the module selection part 10B determines that the proposed medical treatment module merely produces a small effect, and selects and proposes a new medical treatment module. The medical treatment module part 10C transmits (provides) the new medical treatment module to the patient, and the evaluation part 10D evaluates a therapeutic effect based on the new medical treatment module.

In Fig. 2, the patient-side information terminal 3 is connected to the diagnostic cross-sectional assessment part 10A1 through the information transmission line SL12. As described with reference to Fig. 1, the patient-side information processing terminal 3 has a function for measuring patient's "normal lifestyle data" and transmitting the measured normal lifestyle data to the diagnostic cross-sectional assessment part 10A1. In order to carry out such the function, the patient-side information processing terminal 3 transmits the patient's "normal lifestyle data " to the diagnostic cross-sectional assessment part 10A1 through the information transmission line SL12.

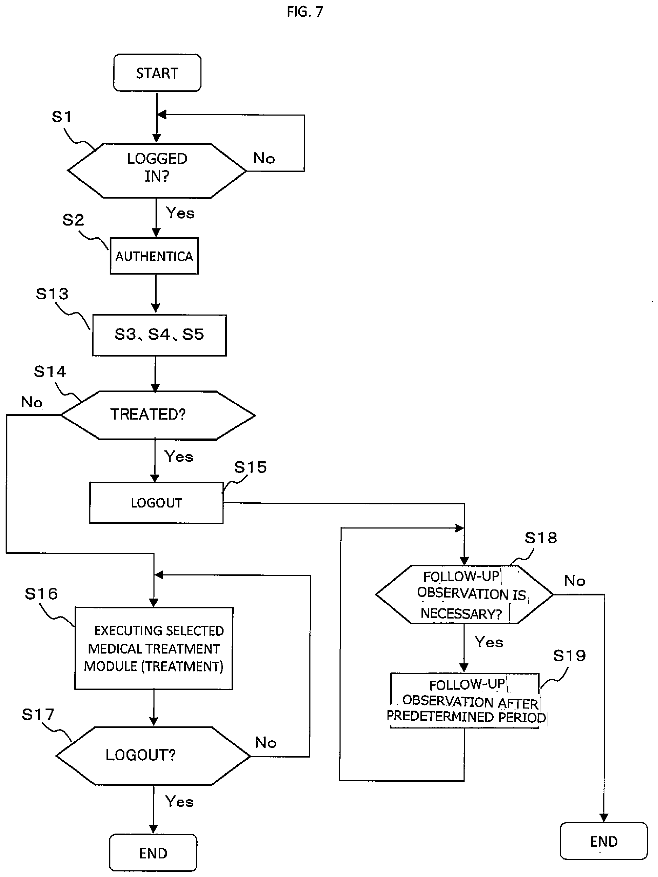

Moreover, the login/authentication part 10H is connected to the medical treatment module part 10C through the information transmission line SL12 and is connected to the evaluation part 10D through the information transmission line SL13. Because, as described later with reference to Fig. 7, in the second and subsequent accesses from the patient (subject), there are cases one of which cases the patient accesses to the medical treatment module part 10C first and executes the medical treatment module, and another one of which cases the patient accesses to the evaluation part 10D to confirm whether a symptom and/or situation of the patient is improved.

Next, the module selection part 10B will be described with reference to Fig. 3.

In the illustrated embodiment in which a platform is constructed so as to include both the diagnostic cross-sectional assessment part 10A1 and the disease-specific assessment part 10A2, the module selection part 10B selects some medical treatment modules on the basis of a result of the assessment performed in the diagnostic cross-sectional assessment part 10A1, and also selects some medical treatment modules on the basis of a result of the assessment performed in the disease-specific assessment part 10A2. In other words, a plurality of medical treatment modules is selected in the module selection part 10B on the basis of the result of the assessment performed in each of the diagnostic cross-sectional assessment part 10A1 and the disease-specific assessment part 10A2.

In Fig. 3, the module selection part 10B comprises an analysis block 10B1, a classification block 10B2, a linking block 10B3, and a storage device 10B4.

A result of the assessment performed in the diagnostic cross-sectional assessment part 10A1 is transmitted to the analysis block 10B1 in the module selection part 10B through the information transmission line SL5. Then, a result of the assessment performed in the disease-specific assessment part 10A2 is transmitted to the analysis block 10B1 through the information transmission line SL4.

The analysis block 10B1 has a function for carrying out a cluster analysis with regard to each of the assessment results of the diagnostic cross-sectional assessment part 10A1 and the disease-specific assessment part 10A2.

An analysis result of the analysis block 10B1, which result is based on the result of the assessment performed in the diagnostic cross-sectional assessment part 10A1, is transmitted to the classification block 10B2 through the information transmission line SL22, and an analysis result of the analysis block 10B1, which result is based on the result of the assessment performed in the disease-specific assessment part 10A2, is transmitted to the classification block 10B2 through the information transmission line SL24.

The classification block 10B2 classifies the patient into a predetermined cluster on the basis of the patient's result of the assessment performed in the diagnostic cross-sectional assessment part 10A1. Similarly, the patient is classified into a predetermined cluster on the basis of the patient's result of the assessment performed in the disease-specific assessment part 10A2.

Information of the cluster classified on the basis of the result of the assessment performed in the diagnostic cross-sectional assessment part 10A1 is transmitted to the linking block 10B3 through the information transmission line SL26, and also, information on the cluster classified on the basis of the result of the assessment performed in the disease-specific assessment part 10A2 is transmitted to the linking block 10B3 through the information transmission line SL28.

Information for linking the cluster and a medical treatment module being effective for such the cluster is stored in the storage device 40. The "information for linking" may be a table or a function for associating the cluster and the medical treatment module being effective for such the cluster.

The "information for linking" is transferred to the linking block 10B3 through the information transmission line SL30, and it is determined a medical treatment module being effective for the cluster which is classified in the classification block 10B2. In other words, the linking block 10B3 has a function for determining the medical treatment module effective for the cluster in which the patient is classified in accordance with the "information for linking".

In the linking block 10B3, it is determined that a medical treatment module being effective for the cluster classified on the basis of the result of the assessment performed in the diagnostic cross-sectional assessment part 10A1 and a medical treatment module being effective for the cluster classified on the basis of the result of the assessment performed in the disease-specific assessment part 10A2.

The medical treatment module being effective for the cluster classified on the basis of the result of the assessment performed in the diagnostic cross-sectional assessment part 10A1 is transmitted to the medical treatment module part 10C through the information transmission line SL61, and the medical treatment module being effective for the cluster classified on the basis of the result of the assessment performed in the disease-specific assessment part 10A2 is transmitted to the medical treatment module part 10C through the information transmission line SL62. Consequently, it is possible to propose both the medical treatment module based on the assessment results of the diagnostic cross-sectional assessment part 10A1 and the disease-specific assessment part 10A2 to the patient. In other words, it is possible to perform both the diagnostic cross-sectional intervention and the disease-specific intervention.

The information transmission lines SL61 and SL62 shown in Fig. 3 are comprehensively shown as a single information transmission line SL6 in Fig. 2.

Although the cluster analysis is written as an example of a statistical method, statistical methods except for the cluster analysis can also be applied if the patient (subject) is accurately classified and linked to the appropriate medical treatment module by said statistical methods except for the cluster analysis.

As described above, it is possible to propose both the medical treatment module based on the result of the assessment performed in the diagnostic cross-sectional assessment part 10A1 and the medical treatment module based on the result of the assessment performed in the disease-specific assessment part 10A2 to the patient. However, it can be constructed so as to displayed one of the above-described medical treatment modules in priority against to other one on the patient-side information terminal 3, and also, it can be constructed so as to display both modules simultaneously on the patient-side information terminal 3, case by case. It can be constructed so as to set selectively.

For example, in Fig. 3, there is provided a priority determination block 10R being constructed to input a result of the medical interview and the input performed in the question part 10Q, a result of the assessment performed in the disease-specific assessment part 10A2, a result of the assessment performed in the diagnostic cross-sectional assessment part 10A1, and the like. The priority determination block 10R has a function for determining, on the basis of the input information, whether the medical treatment module based on the result of the assessment performed in the diagnostic cross-sectional assessment part 10A1 should be presented to the patient in priority, or the medical treatment module based on the result of the assessment performed in the disease-specific assessment part 10A2 should be presented to the patient in priority, or both of said medical treatment modules should be presented to the patient simultaneously. In addition, the priority determination block 10R has a function for transmitting a control signal to an interface 10BIF in the module selection part 10B through the information transmission line SL32, in order to control whether transmit the medical treatment module based on the assessment result of the diagnostic cross-sectional assessment part 10A1 to the patient-side information terminal 3, or transmit the medical treatment module based on the assessment result of the disease-specific assessment part 10A2 to the patient-side information terminal 3.

Consequently, it is possible to determine appropriately which should be informed or transmitted to the patient-side information terminal 3, the medical treatment module based on the assessment of a diagnostic cross-sectional assessment part 10A1, the medical treatment module based on the result of the assessment of the disease-specific assessment part 10A2, or both of the above-mentioned medical treatment modules simultaneously.

In the illustrated embodiment, in order to promote a patient who is an application user continuing to use the application and to prevent dropping out during the medical treatment, the system 100 has a function for sending a message (reminder notification) encouraging the patient make login to the digital therapeutics (medical treatment application) at an appropriate timing from side thereof to the patient (function for activating the application at an appropriate timing).

For example, it is possible to obtain the normal lifestyle data of a patient who uses the system 100, immediately activate the medical treatment applications, and perform the assessment real time on the basis of the normal lifestyle data. It is also possible to intervene immediately (real-time intervention) as a result of such the assessment performed in real time.

Not only a determination on the basis of the numerical values of the normal lifestyle data is carried out, when an event by which the patient expects to require the medical treatment applications is happened in patient's normal life, the digital therapeutics (medical treatment applications) can also be activated to perform assessments and take other necessary measures. For example, in a case that a psychogenic ED patient is using the medical treatment applications keywords and schedule in e-mail received by the patient are checked. Then, for example, if e-mail offering a dating is dispatched from a partner, it determines that an event requiring the medical treatment application is happened, the medical treatment application is activated, a required assessment is executed to transmit a message encouraging to execute the medical treatment module required for the patient.

In a case that a patient is a chronicity pain patient, it can detect that the patient has received "picnic invitation e-mail", the digital therapeutics (medical treatment applications) can be activated, a required assessment can be executed, and a message can be transmitted from the system 100 side, or a message encouraging to execute the medical treatment module being required for the patient can be transmitted.

By feeding back to a therapist from the normal lifestyle data and patient's other data,
as regards "At what point (timing) does the patient have a problem?",
it can be determined, for example,
"When does the patient have a problem, morning, noon, or night?'
or "Will a given case cause problems for the patient?". That is, by further analyzing the data automatically collected by the system according to the illustrated embodiment, it is possible to determine timing at which a patient has a problem, in other words, timing at which the patient most needs the treatment application.

Thus, determining the appropriate timing to activate the digital therapeutics (medical treatment applications) and preparing the necessary measure in the digital therapeutics (medical treatment applications) means to provide a function for immediately executing the assessment for the patient when the patient urgently needs the medical treatment module to be executed (i.e., an emergency for the patient), or a function so that the patient can immediately execute the medical treatment module and Tips (so-called "rescue-like" function).

As such rescue measures and functions, it can be considered to display the contact phone number of a medical doctor who prescribes the necessary PDE5 inhibitor, for example, if the patient is in the ED and has been contacted by the partner as described above but the patient has no PDE5 inhibitor.

Then, a rescue button is provided on the patient-side information processing terminal 3, said button has a function that if the patient operates the rescue button in a case that the above-described situation occurs, required medical treatment module, exercise, information, and the like is immediately presented to the patient from the digital therapeutics side (medical treatment applications side). In a case that the patient is in the ED and has been contacted from the partner but the patient has no PDE5 inhibitor, it is considered to pop up countermeasures previously considered (countermeasures that the patient himself/herself had determined) in the medical treatment module.

Then, in a case that the rescue button is provided on the patient-side information processing terminal 3, if the patient operates the rescue button in the above-described situation, required medical treatment module, exercise, information, and the like is immediately presented to the patient from the medical treatment applications side. If the patient is in the ED and has been contacted by the partner but the patient has no PDE5 inhibitor, in such a case, the patient falls into a so-called "State of Drowning in Thoughts" due to anxiety such as "What if my partner hates me?" or "What if my sexual intercourse fails?"

In the illustrated embodiment, if the medical treatment module and the exercise are transmitted to the patient and then the patient can also think that "sexual activity is not everything" and "simply enjoy meeting my partner", the patient can get out of the anxious, "state of drowning in thoughts".

Although not shown in drawings, in the system 100, it is possible to provide with a device having a function for transmitting messages periodically or irregularly, such as reminder notification and encouragement, encouraging to use the medical treatment applications, at a regularly determined time. It is also possible to apply gaming elements. Furthermore, it is possible to provide a function for providing some reward to the patient for accessing the system 100. The system can be provided with a wearable device or the like having the above-described functions to increase motivation for accessing the system 100 and executing the medical treatment applications actively, thereby reinforcing the "positive" behavior of continuing the medical treatment (continuing the medical treatment applications successionally).

By exerting the above-mentioned functions, it is possible to motivate the patient to continue to access the system 100 and execute the medical treatment module, and is possible to prevent a situation where the patient stops accessing the system 100 in the middle of the medical treatment (dropout in the middle of the medical treatment) and to improve the therapeutic effect produced by the system 100 (or effect of the medical treatment applications).

The timing of dispatching the reminder notification can be determined by managing information relating to "whether the patient is active or inactive" and information relating to "whether the patient is moving or being at home"(status management).

Moreover, on the basis of the normal lifestyle data, reminder notification can be dispatched at the time when the heart rate is stable, when the patient is not busy, or the like.

Furthermore, since there is a high possibility that the patient touches the smart phone after the patient-side information processing terminal 3 (e.g., a smart phone) is activated (after resumption), the reminder notification can be sent out at such the time.

In the illustrated embodiment, in order to promote the patient continuing to use the application (medical treatment applications) and to prevent dropping out during the medical treatment, it is possible to provide contents for the patient oneself to strengthen an intention of engaging in the medical treatment and an intention of participating in the medical treatment oneself (to increase adherence).

Such contents include contents relating to disease education, treatment protocol education, and the like (e.g., a content for explaining ACT, Acceptance and Commitment Therapy).

In order to maintain the patient's motivation to continue using the application and to prevent the patient dropping out in the middle of the medical treatment, it is important to make the patient log in to the digital therapeutics (the medical treatment applications). For this purpose, in the illustrated embodiment, a reminder notification (request to log in to the medical treatment application) is sent (dispatched) when the patient accesses to a social networking service (SNS) application (e.g., "Twitter" (registered trademark: an information service provided by Twitter Inc.) of a celebrity that the patient likes.

Alternatively, the reminder notification is dispatched when the patient accesses the partner's SNS application (e.g., "LINE (registered trademark: an information service provided by LINE Corporation)".

Furthermore, on the basis of the activate situation of other SNS applications (e.g., "YouTube (registered trademark: a video sharing service provided by Google LLC.)" etc.) for the smart phone used by the patient, it is possible to send (dispatch) the reminder notification linked thereto.

It has been found that an alarm (alert) generated from the patient-side information processing terminal 3 (e.g., smart phone) raises awareness of the issues of the patient and improves the patient motivation for continuation of the medical treatment.

For example, in a case that a patient has ED, diabetes can be a complication. In the illustrated embodiment, by measuring the patient's blood glucose level on a daily basis and generating an alert when the blood glucose level exceeds a predetermined value, it is possible to give the patient a sense of crisis and increase the motivation for continuation of the medical treatment. As mentioned above, in the illustrated embodiment, since the diagnostic cross-sectional assessment is carried out, it is effective in a case that a plurality of diseases (diabetes, arteriosclerosis, and the like) are associated with one another as above-mentioned.

Moreover, focusing on the fact that meals (appetite) are related to mood, and that appetite changes (decreases or increases) during depression, it is possible to create the system so that the patient oneself can recognize to be depressed by an appetite score indicating changes in appetite. Alternatively, it is possible to create the system so that the patient oneself can recognize to be depressed by uploading a photograph of a meal and being able to view the change of appetite.
Alternatively, a photograph of a meal is uploaded and change of appetite can be viewed, thereby recognizing to be depressed.

There is one theory in behavior analysis regarding the "positive behavioral reinforcement" mentioned above, by which theory the presence of a "good thing" or "disappearance of an unpleasant thing" (reward) being generated immediately after the behavior operates in the direction of continuing the behavior. The "immediately after" used herein is within 60 seconds, and this theory is sometimes expressed as the "60-second rule". Based on this theory, in the illustrated embodiment, it is possible to motivate the patient to continue the behavior (medical treatment) by feeding back "You tried hard!" or the like, adding points, displaying the degree of achievement, displaying the result in a graph, and the like, immediately after the medical treatment module or exercise is executed or carried out.

Although not clearly shown in drawings, in the illustrated embodiment, the design/visual of the digital therapeutics (medical treatment applications) can be constructed such that, for example, the background design can be selected in order to be able to activate due to the effect of visual information by merely looking.

Moreover, patient's respect for the digital therapeutics (medical treatment applications) contributes to the continuation of using the application. Accordingly, it is appropriate that the design of the medical treatment application according to the illustrated embodiment should not be realized by around other people. For example, it is preferable to have a design that makes a third party looks like the patient with psychogenic ED is looking at patient's smartphone for business (even though the patient is running the ED treatment application) so that the patient will not feel embarrassed if a third party sees the patient while the patient is running the medical treatment application. Such a design etc., can solve the problem of the patient being discouraged from continuing with the treatment application because of "embarrassment (if other persons know that the patient is running the medical treatment application)". Then, for example, since the medical treatment of ED can be executed in spare times except for at home, it becomes easy for the patient to continue the medical treatment using the medical treatment applications.

Upon carrying out the diagnostic cross-sectional assessment, the disease-specific assessment, the medical treatment module (medical treatment program: exercise, work), homework, Tips, evaluation, and the like, it is possible to be constructed to provide a voice interaction function (interactive function) by permitting patients to input, record, and respond with voice in response to read-out of question items, instructions, guidance, and the like with voice by using a smart speaker or the like. The smart speaker (AI speaker) used herein is a speaker with AI assistant function that supports interactive voice operation, recognizes a voice by means of a built-in microphone, searches for information, and operates linked home appliances.

Moreover, it is possible to provide with a device having a function for providing contents (Tips) to the patient accessing to the system 100 sequentially, said contents include view/listen videos, music, sounds, and other contents (e.g., mindfulness meditation) and relate to the medical treatment or useful for medical treatment. Such contents may include contents, for example, mindfulness meditation, an aroma, music, videos, vibrations, and information on preferred foods/dietary items, etc., and improves the index so-called QOL by means of using the five senses. The index QOL indicates therapeutic effects, continuation thereof, and satisfaction.

Upon executing the above-mentioned medical treatment module and the exercises, it is possible to combine with VR and AR when moving the body according to the medical treatment application, and to wear VR and AR equipment when executing the exercise, thereby enhancing the real feeling by the VR and AR. Consequently, the effect of the medical treatment module and the exercise can be improved.

Here, when executing the medical treatment application, it is preferable to perform the work together with the patient's partner as the woman, and to watch the contents with the partner, because raising the partner's (woman's) awareness and promoting the partner's understanding and transport to ED treatment.

In the illustrated embodiment, it is constructed so that a brief evaluation (mini evaluation) is carried out for every exercise of the medical treatment module, e.g., a patient's oneself evaluation can be written in a "free description field (free text field)". By feeding back of such a mini evaluation and changing patient recognition in the feeding back, it is possible to shown the patient the significance of reaching the goal (e.g., the significance of executing the exercises every day). If such significance can be shown, for example, for a patient with psychogenic ED, a hope that " even if sexual activity is not going well, progress is being made, and it is improving more than before" can be given (to the patient).

In addition, by making the patient feel a sense of the significance with regard to the feedback and by carrying out a feeding back for working toward the goal, the patient's motivation of continuation of using the medical treatment application will be able to be improved.

Here, the illustrated embodiment focuses on the "process" rather than the "result". For example, in the case of a patient with psychogenic ED, the focus is on whether or not the psychological "process" stage of the patient has progressed and thereby the patient's how to use of the mind has improved, rather than whether or not the symptom of "erection or lack of erection" has improved. If the stage of the psychological process progresses and the patient's way of thinking improves, it means that a new behavioral pattern has been acquired and learned. Accordingly, in the above-described case, a range of the way of thinking of the patient with the psychogenic ED can be expanded so that the patient can consider such as "it's okay if cannot get an erection" and "the relationship with my partner is not all about sexual activity".

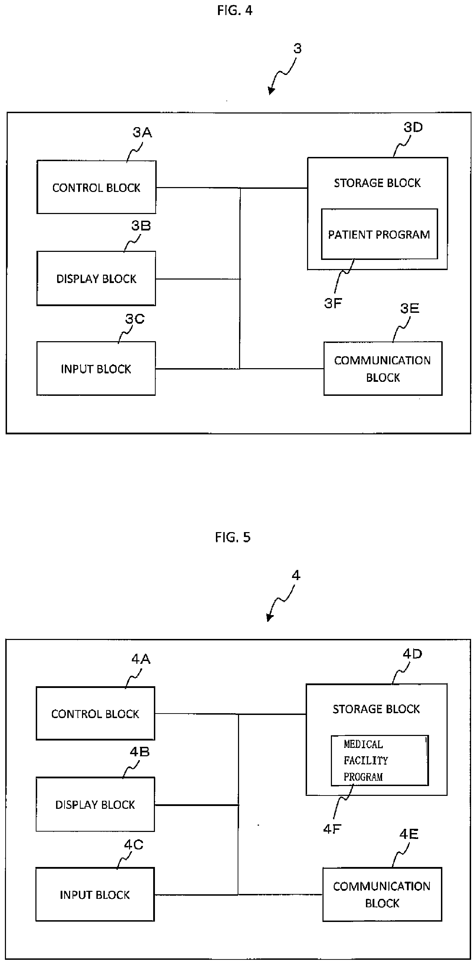

In Fig. 4, the patient-side information processing terminal 3 comprises a control block 3A, a display block 3B, input block 3C, storage block 3D, and a communication block 3E.

The control block 3A has a function for controlling information processing and the like in the patient-side information processing terminal 3. The display block 3B has a function for displaying information to a user (patient) using the patient-side information processing terminal 3. Input block 3C has a function for receiving information being input by the user (patient).

The storage block 3D is constructed by an already-known storage device, and a patient program is stored shown by the reference character 3F which program is used for the patient-side information processing terminal 3. Although not clearly shown in drawings, it is possible to use the database 10F as the storage block 3D. The communication block 3E has a function for connecting to the network 20 through wired or wireless communication.

The patient-side information processing terminal 3 is not particularly limited, as long as electronic equipment having an information processing function and a communication function. However, in consideration of recently spread thereof, it is preferable to be constructed by a personal digital assistant (portable information terminal) such as the so-called smart phones.

In Fig. 5, the medical facility-side information processing terminal 4 comprises a control block 4A, a display block 4B, an input block 4C, a storage block 4D, and a communication block 4E.

The control block 4A has a function for controlling information processing, etc. in the medical facility-side information processing terminal 4. The display block 4B has a function for displaying information to users (medical doctor, staffs of medical facility, and the like) who uses the medical facility-side information processing terminal 4. The input block 4C has a function for receiving information being input by the medical doctor, the staff of medical facility, and the like.

The storage block 4D is constructed by an already-known storage device, and a medical facility program shown by the reference character 4F is stored which program is used for the medical facility-side information processing terminal 4. It is also possible to use the database 10F as the storage block 4D. The communication block 4E has a function for connecting to the network 20 through wired or wireless communication.

Although not clearly shown in drawings, it is possible to connect to a website being outside of the system, such as an anonymous patient party, a chat room and BBS, so that a community of patients suffering from the same disease can share usage and progression of the medical treatment application and view the date of other users as statistical data. Here, it is necessary for the websites outside of the system being able to be connected to satisfy requirements as a medical device.

By connecting to such websites being outside of the system, it is possible to obtain an effect being equivalent to the effect which can be obtained by participating in a self-help group for various diseases (e.g., self-help group for drug addicts), and to improve the motivation of the patient for to continue the medical treatment.

By connecting to such websites being outside of the system, it is possible to obtain an effect equivalent to participating in a self-help group for various diseases (e.g., self-help group for drug addicts), which can improve the motivation of the patient to continue the medical treatment.

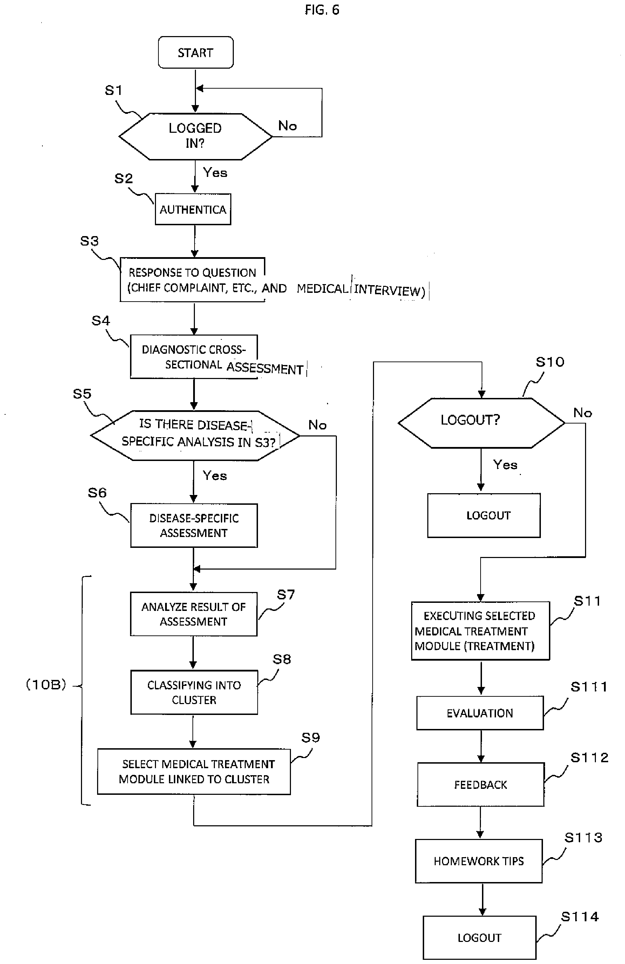

Next, mainly referring to Figs. 6 and 7, procedures for carrying out diagnosis, medical treatment, prevention, and the like (medical care) by the system 100 will be described. In explanations in reference to Figs. 6 and 7, it is assumed that the disease of a patient (person concerned; user) is psychogenic ED.

First, referring to Fig. 6, in a case that a patient accesses the system 100 at the first time will be described.

In Fig. 6, it is determined in Step S1 whether or not the patient-side information processing terminal 3 has logged in the system 100, thereby determining whether the patient uses the system 100. In Step S1, if there is "login" from the patient side ("Yes" in Step S1), the process proceeds to Step S2, and if there is not "login", the process returns to Step S1 (loop in the case of "No" in Step S1).

Although not clearly shown in Fig. 6, in Step S1, the login/authentication part 10H verifies identity of accessing patient, for example, by means of an issued ID and password. Although not shown in drawings, it is also possible to determine appropriately the number of times of accessing the system and the period of time (treatment period) since the first access, for a patient who has accessed the system multiple times, and to deactivate or restrict the access of such the patient if the set number of times or the set period of time is exceeded to the determined ones.

Although not shown in drawings, at the time of login authentication, in addition to input of the ID and the password, in a case that the patient-side information processing terminal 3 is a smart phone or a PC, for example, biometric information being unique to the patient, such as the patient's face, voice (voiceprint), retina, and iris, can be captured (obtained) using a camera of the terminal, and authentication of the patient can be performed by utilizing such the biological information. It is possible to obtain the above-mentioned biological information not only at the time of login but also any time of inputting (obtaining) patient's data. By obtaining of said biological information and carrying out authentication of the patient, it is possible to prevent a behavior so-called "impersonation" and account transfer, and thereby, to ensure the reliability of data being input or being obtained from the patient.

In Step S2 (when logged in by the patient), the login/authentication part 10H authenticates the patient on the basis of the ID and the password input by the patient side. Then, the process proceeds to Step S3.

Although not clearly shown in Fig. 6, the patient authenticated in Step S2 can proceed to Step S113 (described later) immediately, without carrying out procedures of Step S3 and subsequent steps.

In Step S3, the accessing patient should input the chief complaint, the current medical history, the past medical history (intake information) and the like, and also, the psychogenic ED evaluation and the medical interview based on the operative diagnostic criteria are carried out. Here, the psychogenic ED evaluation relates to thoughts (e.g., "when it begins to consider that it will end in failure moreover, the head will fulfill with that" and "I can't concentrate on sexual activity because I worry about whether I'll be able to maintain an erection") and the medical interview relates to questions (e.g., questions in the form of checklist by DSM-5 and ICD-10 compliant psychiatric simplified structured interview method). Then, the process proceeds to Step S4.

In Step S4, the diagnostic cross-sectional assessment part 10A1 assesses the degree to how the patient is adaptively behaving or non-adaptively behaving in response to psychosocial issues, lifestyle adaptation, stress and the like, by utilizing normal lifestyle data, and thereby, the psychological flexibility of the accessing user (patient: subject) is assessed (diagnostic cross-sectional assessment). In the illustrated embodiment, a performance of the diagnostic cross-sectional assessment is carried out in priority to a performance of the disease-specific assessment.

After the diagnostic cross-sectional assessment performed in Step S4, in Step S5, the presence or absence of disease-specific signs is determined as a result of the input and the medical interview executed in Step S3. If there are no disease-specific signs, the process proceeds to Step S7 ("NO" in Step S5: e.g., in the case of healthy person).

On the other hand, if there are disease-specific signs ("Yes" in Step S5), the process proceeds to Step S6.

In Step S6, the disease-specific assessment part 10A2 assesses whether the above-described skills are demonstrated for the specified disease (disease-specific assessment).

After the disease-specific assessment performed in Step S6, the process proceeds to Step S7.

If it is determined that the subject is a healthy person in Step S3, the process immediately proceeds to Step S7, and it is also possible to classify the subject into a cluster corresponding to the healthy persons.

In Step S7, the analysis block 10B1 carries out (for example) a cluster analysis with respect to the result of the diagnostic cross-sectional assessment, or carries out (for example) a cluster analysis with respect to a result of the disease-specific assessment.

Then, in Step S8, based on the result of the cluster analysis, the classification block 10B2 classifies the patient into the corresponding cluster on the basis of the cluster analysis of the diagnostic cross-sectional assessment, and classifies the patient into the corresponding cluster on the basis of the cluster analysis of the disease-specific assessment.

In the diagnostic cross-sectional assessment or the disease-specific assessment, nine evaluation indices are conventionally used in the case of psychogenic ED, but as a result of the study by the present inventors, it is found that the cluster analysis in the case of the psychogenic ED can be carried out by using only three indices. The three indices are AFQ-ED "Psychological Flexibility in ED (Avoidance and Fusion Questionnaire-Erectile Dysfunction)", SRQ "Sexual Communication with Partner (Communicating with Partner About Sexuality)", and PFQ "General Psychological Flexibility (Overall Psychological Flexibility)".

After classifying the patient into the corresponding cluster, the linking block 10B3 determines (links) a medical treatment module being effective for the classified cluster of the patient (Step S9). Then, the process proceeds to Step S10.

Steps S7 to S9 are executed in the module selection part 10B.

In Step S10, the patient (the medical treatment module thereof has been determined) determines whether he (or she) will log out of the system 100 at this time point.

If the patient is satisfied with the presentation of the medical treatment module as the first access ("YES" in Step S10), then the patient logs out, and the first access for the patient is completed. In a case that the patient accesses again (second and subsequent accesses are carried out), it will be described later with reference to Fig. 7.

If the patient requests not only the presentation of the medical treatment module but also execution of the presented medical treatment module ("No" in Step S10), the medical treatment module selected by the module selection part 10B (Step S11) is carried out (executed) at the medical treatment module part 10C. The manners of such the execution will be different depending on the content of the medical treatment module.

After the execution, the process returns to Step S10.

Although not shown in drawings, in Step S11 (and Step S16, which will be described later with reference to Fig. 7), a part of the medical treatment program (exercise, work, etc.), homework, and Tips of the medical treatment module, which are shown corresponding to the patient's cluster, can be transmitted to the patient-side terminal 3. By transmitting the part of the medical treatment program, the homework and the Tips to the patient-side terminal 3, the patient can carry out the medical treatment program at a location without network environment or off-line. Accordingly, for example, even if a problem is happened in communication/network environment or even if it becomes impossible to access a system 100 (if it is non-activated) after medical treatment is completed, the patient can continue to carry out and browse subsequently a part of exercise and work (e.g., voice for attention training, etc.), homework, and information (Tips), which were performed in the medical treatment module, in off-line manner, and thereby, the patient can be prevented to interrupt the medical treatment and can be promoted to continue the therapeutic effect and to prevent recurrence.

In order to prevent a situation in which it is impossible to evaluate appropriately because of the exercise and the workout, homework, and Tips which were performed in off-line manner are not recorded as an intervention record in the system 100 side, it is possible to transmit an information of the exercise and the workout, which are performed in off-line manner, to the system 100.

It is possible to construct so that recording of the executed medical treatment module can be input by the voice upon carrying out the medical treatment module.

After the medical treatment module is carried out (Step S11), by using the diagnostic cross-sectional (psychological flexibility) measurement index, the disease cross-sectional measurement index, the already-known evaluation scale and the like, an effect of intervention is measured (Step S111: evaluation). After the effect of intervention is measured, a feeding back is provided to the medical doctor and the patient (Step S112). Upon said feeding back, the patient's normal lifestyle data can be obtained and recorded by using the patient-side terminal 3, the wearable device or the like. Thereby, it possible to indicate the results of the effect measurement before and after the intervention in a report format such as graphs, and to provide the feeding back to the medical doctor and the patient. Then, for each predetermined number of times or period of time, a report can be prepared and indicated to the medical doctor and the patient. Said report make understand the medical treatment module and the resulting changes thereof (changes in evaluation indices due to the intervention, changes in the normal lifestyle data , and the like).

In the feeding back (step S112), it is possible to transmit a message such as "You did XX in the last work and exercise" from the medical treatment application side to the patient. The patient who received such a message have the impressions "The app has not forgotten about me" and an "I'm getting attention", and such impressions can increase the motivation of the patient to continue the medical treatment application.

Moreover, in the feeding back (Step S112), a degree of patient's emotion or stress can be determined objectively by using an emotion recognition AI based on facial expressions.

Furthermore, if the patient wears earphones with a built-in pulse sensor, body temperature sensor and the like, even in an environment in which the patient cannot hear voice due to noise, the normal lifestyle data, especially a pulse, which is a normal lifestyle data detected by voice and vibration, can be obtained. Similarly, body temperature data can also be obtained. Upon feeding back, it is possible to determine the patient's emotional change and stress on the basis of the pulse rate data and the temperature data obtained in this manner.

In the illustrated embodiment, it is possible to visualize degrees of "psychological flexibility" and "mindfulness" in Acceptance and Commitment Therapy (ACT) by utilizing the normal lifestyle data.

As the normal lifestyle data, for example, a sensor can be attached to the patient's chest to measure the heart rate and calculate the parameter LF/HF (the power ratio of low frequency LF and high frequency HF: indicating the overall balance between sympathetic and parasympathetic nerves) which parameter indicates a balance of autonomic nerves functions, and thereby the autonomic nerve state can be indicated. Here, as the sensor to be attached to the patient's chest, any known and commercially available sensor can be used. The data measured by the sensor is transmitted as a data to the patient-side information processing terminal 3 and stored therein. By comparing LF/HF calculated by the measured heart rate data with the tendency of LF/HF in the patient, it is possible to determine whether there is an abnormality in the patient's autonomic nerves balance at the time carrying out the comparison and to confirm the effect of the medical treatment.

Moreover, as the normal lifestyle data, hormone/stress levels can be measured and used as an evaluation index of an effect of the medical treatment. For example, cortisol and amylase in saliva are measured by a sensor and transmitted the measurement result thereof to the patient-side information processing terminal 3 as a data. Alternatively, the blood concentration of dopamine or serotonin can be measured by a sensor and transmitted the measurement result thereof to the patient-side information processing terminal 3 to be used as an index of the effect of the medical treatment. The serotonin can also be measured by identifying alpha waves with an electroencephalograph sensor.

Here, cerebral blood flow can be measured instead of electroencephalogram, as the normal lifestyle data. For example, since overactivity of the right frontal pole is relating to the self-attention, it is possible to define an index of anticipatory anxiety and self-attention by measuring brain activity in the right frontal pole through a cerebral blood flow (NIRS: Near Infra- Red Spectroscopy) sensor, and thereby, it is possible to detect the station in real time in which station the patient is overly self-conscious.

Furthermore, an amount of communication, which is a physical quantity and the amount of information, such as the number and frequency of conversations, the time spent in conversation, an amount of information in conversation, can be measured by a smart phone (PC or a tablet-type device can also measure such the physical quantity) and used as the normal lifestyle data. As a method of measuring the amount of communication, it is also possible to detect an IC chip embedded in a device such as a smart phone by a detecting means, and then, to measure the number of times automatically at which time the detecting means is in proximity to the IC chip and measure time period automatically in which period the detecting means is in proximity to the IC chip.

By obtaining and accumulating digital biomarkers, which are physiological data that can be obtained by wearable devices, etc. as the normal lifestyle data, it is possible to utilize the obtained data so as to predict the onset of disease, to understand and manage conditions, etc., and to improve the accuracy of assessments. Then, more personalized and optimized medical treatment can be proposed/provided. Furthermore, it is possible to be a big data being utilizing in the development of algorithms using machine learning and AI.

By utilizing this big data and, for example, by utilizing AI, it is possible to get predictions relating to diseases, and therefore, it is also possible to construct preventive measures on the basis of such the prediction. Then, interventions are possible for preventing disease. Alternatively, it is possible to analyze big data by AI, and to provide the medical treatment application on the basis of a result of the analysis by AI, a content of which medical treatment application adapts to the individuality of each patient, the degree of disease and the like (i.e., said content is suitable for each patient). That is, it is possible to provide an application (a medical treatment application) being customized for each patient.

Here, the patient-side information processing terminals 3 (e.g., smart phone, PC, tablet-type device, wearable device, or the like) can have a function for recording the normal lifestyle data, which function is as like as a function of "life record table". In prior arts, patients have recorded the normal lifestyle data on paper media. However, by electronically recording the normal lifestyle data on the "life record table", due to the simplicity of operating smartphones, PCs, tablet-type devices, wearable devices and the like, it is possible to record the normal lifestyle data in real time. In a case that the data is recorded on paper, there is a tendency that the normal lifestyle data in a certain period of time is recorded collectively at the time point that said certain period of time elapses, and as a result, there is a possibility of bias (i.e., the possibility of filling in the paper media with content which is different from true fact or an fact actually felt). By electronically recording the normal lifestyle data in real time on the patient-side information processing terminal 3, such the biases and false memory recall can be prevented, and then, more appropriate and valid normal lifestyle data can be stored rather than the case of paper-based recording.

As mentioned above, in the present invention, it is possible to visualize a degree of "psychological flexibility" and "mindfulness" in Acceptance and Commitment Therapy (ACT) by utilizing the normal lifestyle data. An example of psychological data in the normal lifestyle data, for example, patient's responses is used. Said patient's responses is executed in a manner that the Ecological Momentary Assessment (EMA) method is used, in which manner the patient answers in accordance with their moods, feelings and thoughts, at every morning and every night, on the spot, at that time, in that moment. By making the patient respond on the spot, at that time, in that moment (i.e., respond in real time), it is possible to prevent bias in their memories. Here, the "every morning and every evening" is used as an example of the timing of the patient's responses, but it is not limited to a fixed time. Reminder notifications can be sent (dispatched) randomly within a certain time period (e.g., 7:00 to 8:00 in the morning and 19:00 to 22:00 in the evening) in order to encourage responses.

Also, the method of an Ecological Momentary Assessment (EMA) is applied to physiological data and behavioral data, such as a heart rate, blood pressure, blood oxygen level, the number of steps, sleep time and sleep quality (deep sleep, shallow sleep, and the like), active mass (activity), an activity range (an activity history of behavior history: e.g., measured and obtained by GPS), and physiological data such as blood glucose levels.

Upon the evaluation in Step S111, for example, the patient inputs the response of the measurement index (effect of intervention). For example, if the patient-side terminal 3 is a smart phone or PC, it is possible to measure the heart rate and the blood pressure, it is possible to measure the state of patient's pupil opening and closing, the direction of the line of sight, and the number of times of the blink by using a camera of the terminal, to measure the number of times of breath and the body temperature, or to observe the complexion, and thereby, it is possible to monitor objective information, at the time until the input is recorded. Thus, by monitoring objectively the accuracy of the response and the condition of the respondent, it is possible to prevent a situation that the patient inputs a measurement index carelessly, a situation that the patient inputs a measurement index falsely in order to obtain an "improvement of result ", or a situation that the patient is subjected to stress. Furthermore, it is possible to measure the heart rate, the blood pressure and the like.

Here, for example, in a case that the patient responds by voice thereof, it is possible to analyze and determine the patient's voice by means of AI and to determine whether the patient is serious about the medical treatment, or it is possible to determine the degree of seriousness of the patient (the patient's level of earnestness).

Although not shown in drawings, by attaching devices such as a smell sensor, an electroencephalogram sensor, and a cerebral blood flow sensor, it is also possible to obtain a respondent's objective information by said sensors and to measure the respondent's physical condition, and thereby, it is possible to utilized the measurement result as the factor of the cluster analysis.

In accordance with the evaluation after the intervention in Step S111, it is possible to instruct the homework to the patient automatically (Step S113). In Step S113, the next intervention (access to the system 100) can be proposed or instructed automatically in accordance with the evaluation after the intervention. Thereby, it is possible to repeat the access until being improved. Moreover, in Step S113, the patient accessing the system 100 can sequentially view and listen video, music, audio, and other contents (e.g., mindfulness meditation) which are related to or useful for the medical treatments (Tips). In the Tips, there are contents, such as mindfulness meditation, aroma, music, video, vibration, and preferable food/meal information. Such contents improves quality of life (QOL) through the use of the five senses. The index QOL indicates a therapeutic effect, its continuation, and a degree of satisfaction.

As described above, it is possible for a patient who have authenticated in Step S2 to proceed immediately to Step S113 and to view and listen the content.

After Step S113, the patient logs out (Step S114). After logging out, when the patient actually goes to the hospital, a medical examination/interview is carried out by a medical doctor, while referring to (by the medical doctor) the intervention, evaluation records and the obtained normal lifestyle data in the system 100.

Next, procedures in a case that second and subsequent accesses will be described with reference to Fig. 7.

Contents of Steps S1 and S2 are the same as mentioned in reference to Fig. 6. As mentioned above, the login/authentication part 10H issues, for example, a prescription ID and a password, and verifies identity of the accessing patient. Although not shown in drawings, it is possible, for example, with the patient's consent, to appropriately set the number of times of accessing to the system 100 and the period of time (treatment period of time) from the first access, and it is possible to deactivate the access if the set number of times or the set period of time is exceeded. Even if the access has been deactivated and thereby access to the system 100 is impossible, with attending doctor's instructions, the patient can access the system 100 again.

Then, the process proceeds to Step S13, and the procedures in Steps S3, S4, and S6 (medical interview, diagnostic cross-sectional assessment, disease-specific assessment) are carried out, which procedures are described with reference to Fig. 6. Then, the process proceeds to Step S14. Although not clearly shown in drawings, the patient authenticated in Step S2 can also immediately proceed to Step S113 (homework, Tips), without carrying out procedures in Step S3 and subsequent steps.

In step S3, the input can be omitted because it is expected to be similar to that for the first access.

In Step S14, with reference to the medical interview, the diagnostic cross-sectional assessment and the disease-specific assessment, the evaluation part 10D determines whether the patient has improved, and measures an effect of previous intervention by means of the diagnostic cross-sectional (psychological flexibility) measurement index and the disease cross-sectional measurement index (the disease-specific measurement index). Also, in Step S14, it is possible to obtain and record the normal lifestyle data by using the patient-side terminal 3, the wearable device or the like, it is possible to express the results of the effect measurement before and after the intervention in a report format such as graphs, and it is possible to provide the feeding back to the medical doctor and the patient.

If the evaluation part 10D determines that it has been improved ("Yes" in Step S14), the process proceeds to Step S15.

If it is determined that it has not improved ("No" in Step S14), the process proceeds to Step S16. In Step S16, the medical treatment module, which was presented at the time of the previous access, is carried out.

Although not clearly shown in Fig. 7, in a case that a patient requests to execute (to carry out) the medical treatment module immediately, as described with reference to Fig. 2, it is possible to skip Steps S13, S14, and S16 and immediately proceed to Step S16 after being authenticated in Step S2.

In a case that the patient is determined to be not improved (No in Step S14), if it is determined that there is no improvement even after repeating the selected medical treatment module for the predetermined time period or the predetermined number of times, the module selection part 10B proposes an other medical treatment module which is selected in the first access.

Alternatively, the module selection part 10B proposes a new medical treatment module on the basis of the diagnostic cross-sectional assessment and the disease-specific assessment in Step S13.

In Step S17, the patient chooses whether he (or she) log out from the system 100. If not logging out, the medical treatment module is continued (loop in the case of "No" in Step S17) .

In Step S15 (in the case of "Yes" in Step S14), the patient logs out of the system 100 and proceeds to Step S18.

In Step S18, for example, the evaluation part 10D determines whether a follow-up observation is necessary. At this time, the evaluation part 10D determines a likelihood of recurrence on the basis of the medical interview, the intake, the diagnostic cross-sectional assessment and the disease-specific assessment. Then, if there is likelihood of recurrence ("Yes" in Step S18), the patient-side information terminal is accessed from the system 100 side after a predetermined period has elapsed, and then, a follow-up observation are carried out through a medical interview of given items and the like (Step S19).

Although not shown in Figs. 6 and 7, it is also possible to transmit messages to the patient periodically or irregularly, such as reminder notification and encouragement, in order to encourage to use the medical treatment application, and it is also possible to apply a gaming element. Furthermore, it is possible to reward the patient in some way for accessing the system 100. It is then possible to increase the motivation to access the system 100, to carry out actively the digital therapeutics (medical treatment applications), and thus, to reinforce the "positive" behavior of continuing the medical treatment (continuously using the digital therapeutics).

Accordingly, it is possible to increase the patient's motivation for continuing use (motivation of accessing the system 100 and continuing to carry out the medical treatment module), it is possible to inhibit a situation in which the patient stops accessing the system 100 in the middle of the medical treatment (dropout in the middle of the medical treatment), and it is possible to improve the therapeutic effect produced from the system 100 (or effect of the medical treatment application).

Although not shown in drawings, in carrying out the diagnostic cross-sectional assessment, the disease-specific assessment, the medical treatment module (medical treatment program: exercise), work, homework, Tips, evaluation, and the like, by using a smart speaker or the like, it is possible to be constructed to provide a voice interaction function (interactive function) by allowing patients to input, record, and respond with voice in response to read-out of question items, instructions, guidance and the like with voice.

In the illustrated embodiment, it is also possible to customize the server 10 in correspondence to diseases, the patient-side application, and the like. Prior arts can be applied to the customization methods.

Although not clearly shown in drawings, in the illustrated embodiment, the patient's medical treatment and behavior such as medication can be recorded, and the medical doctor can check such the record. The record can also be checked through the medical facility-side information processing terminal 4, or can be directly checked by the medical doctor.

In order to record the patient's medical treatment and behavior, in the illustrated embodiment, for example, it is possible to be constructed so that a calendar function is installed in the patient-side information processing terminal 3 and patient's medication (medical treatment) record and behavioral record (e.g., record of sexual activity for ED patient) can be described in each date of the calendar. Then, the medical doctor can check the record described in each date of the calendar. Then, the medical doctor can check the record described in each date of the calendar.

Moreover, in the illustrated embodiment, it is possible to carry out the medical examination (including a case in which the patient goes to a medical facility for medical examination, and a case in which the medical examination is performed by an online function provided in the system 100) by the medical doctor at every predetermined period (e.g., every two weeks), and it is possible to determine the treatment policy, the drug prescription used for the medical treatment and the like in correspondence with the situation of the patient. At this time, it is possible to carry out the assessment (diagnostic cross-sectional assessment, disease-specific assessment) again, it is possible to classify the patient into the specific group based on the cluster analysis again, and it is possible to set newly work and the like to be provided to the patient. Then, it is also possible to utilize a result of the assessment being carried out again for the medical examination.

Without changing the specific group (e.g., cluster 1) to which the patient belongs, the treatment policy, prescription, work content and the like can also be changed in response with changes in the normal lifestyle data and EMA.

It is possible to provide the personalized medical treatment for every patient based on performing the medical examination by the medical doctor for every predetermined period, and based on providing a new work. Consequently, it is possible to provide the optimal medical treatment.

In addition, information of the medical examination executed by the medical doctor, the newly set treatment policy, the drug prescription and the like can be stored in the database 10F or the like.

According to the system 100 of the illustrated embodiment, upon the medical treatment of patient, it is possible to provide the optimal medical treatment module for the patient on the basis of a result of a diagnostic cross-sectional assessment using normal lifestyle data, in which assessment a degree how the patient is behaving adaptively or non-adaptively (a degree to which the patient is behaving adaptively or non-adaptively) in response to psychosocial issues, lifestyle adaptation, stress and the like, and the basis of a result of a disease-specific assessment in which a disease is identified and assesses a degree how the patient is behaving adaptively or non-adaptively (a degree to which the patient is behaving adaptively or non-adaptively) in response to the characteristic symptoms of the disease.

Then, after carrying out the selected medical treatment module, the evaluation part 10D evaluates the therapeutic effect by applying the evaluation scale, and feedback the evaluation result to the patient, and if a favorable evaluation is not evaluated about the therapeutic effect, the evaluation result is transmitted to the module selection part 10B, and a new medical treatment module is selected by the module selection part 10B. For example, if the effect produced by the medical treatment module selected on the basis of the result of the disease-specific assessment is small, it is possible to suggest the medical treatment module selected on the basis of the result of the diagnostic cross-sectional assessment.

Here, it is also possible to obtain and record the patient's normal lifestyle data using the patient-side terminal 3, a wearable device or the like, it is possible to indicate a result of the effect measurement before and after the intervention in a report format on a graphic chart or the like, and it is possible to feed back the report for the medical doctor and the patient.

The illustrated embodiment in which a platform being constructed to comprise both the diagnostic cross-sectional assessment part 10A1 and the disease-specific assessment part 10A2, and in which the "process" is focused on rather than the "result". For example, in the case of a patient with psychogenic ED, the psychological "process" is focused. Said "process" relates to whether stage of the patient has progressed and thereby the patient's use of the mind has improved. Said "process" is focused rather than whether the symptom of "erectile dysfunction has improved. If the stage of the psychological process progresses and the patient's way of thinking improves, it means that a new behavioral pattern has been acquired and learned. Accordingly, in a case of the above-described patient with the psychogenic ED, a range of the way of thinking can be widen, such as "it's okay if cannot get an erection" and "the relationship with my partner is not all about sexual activity". Consequently, the psychogenic ED is cured.

In accordance with the illustrated embodiment, it is possible to improve a certain effect by providing the optimal medical treatment module and thereby performing (carrying out) the medical treatment with the process-based CBT, and then, by customizing the medical treatment for every patient or every type of the diseases. Then, the medical treatment is personalized and optimized by executing the diagnostic cross-sectional assessment and the disease-specific assessment using the quantitative measurement indices and by executing the cluster determination. For example, it can exert therapeutic effects on depression, anxiety, lifestyle disease, chronicity pain, sexual dysfunction, psychogenic erectile dysfunction (ED), various addictions and the like. Moreover, the therapeutic effect can also be exerted even on complications arising from the above-described diseases.

By applying the system 100 of the illustrated embodiment, if the patient is behaving adaptively in response to psychosocial issues, lifestyle adaptation, stress, and the like, blood pressure is stabilized, blood flow is improved, the autonomic nervous system is regulated and immunity is enhanced. Accordingly, the system 100 of the embodiment having the server 10 as described above can be applied also for prevention of infection, such as coronavirus.

Moreover, a higher therapeutic effect can be expected by making combined use of regenerative medicine using stem cells or a medical treatment using a supernatant of the stem cells, and the therapeutic application according to the present invention.

In addition, in accordance with the illustrated embodiment, since the quantitative measurement indices, the objective monitoring methods, and the cluster analysis can be used, it is possible to clearly explain an algorithm of the program to a third party, thereby facilitating the use of actual results as evidence. Accordingly, it becomes easy to prepare data required for various kinds of approval.

Although not shown in drawings, the system or method according to the illustrated embodiment can be used by multiple people together. For example, a partner of a patient of ED or infertility (male or female), a family member and a helper of a patient of depression or anxiety disorder or the like can share information and contents required for medical treatment and the like with the patient, and perform exercises related to the medical treatment module in collaboration with the patient. Thereby, the partner, the family member, the helper, or the like can accompany with the patient, and can deepen their understanding and practice of the disease and how to help. Accordingly, the patient can actively participate in the determination of the treatment policy, and receive medical treatment in accordance with the determination (adherence), and thereby the therapeutic effect can be improved.

It should be noted that the illustrated embodiment is merely exemplification and is not intended to limit the technical scope of the present invention.

For example, in the illustrated embodiment, although psychogenic ED medical treatment is mainly explained, the present invention can be applied to issues of relationship with a spouse or a partner, depression, obsessive-compulsive disorder, panic disorder, agoraphobia, generalized anxiety, social anxiety and speech anxiety, specific phobic disorder, post-traumatic stress disorder, insomnia, irritable bowel syndrome, eating disorder and obesity, obesity and other lifestyle-related diseases, bipolar disorder, borderline personality disorder, attention-deficit / hyperactivity disorder, chronicity or persistent pain, sexual dysfunction, issues of relationship with a spouse or a partner except for erectile dysfunction, alcoholism and other addictions, schizophrenia and other severe psychoses, infertility (male, female), atopic dermatitis, an overactive bladder (urinary incontinence), anti-aging, menopausal disorder, premenstrual syndrome (PMS), psychogenic visual disturbance, dental psychosomatic disease, body dysmorphic disorder, and the like. The present invention can also be applied even complications arising from the above-described diseases.

Moreover, in accordance with the illustrated embodiment, it is also possible to make an assessment in a plurality of complications of disease symptoms/conditions, and to suggest or provide the personalized optimum diagnostic cross-sectional treatment protocol. For example, the psychogenic ED application can assess a tendency of complication, such as depression, anxiety, and diabetes. Alternatively, menopausal disorder can be assessed on the basis of a plurality of indefinite complaints.

### REFERENCE CHARACTERS LIST

- 1: Control unit
- 3: Patient-side information processing terminal (e.g., smart phone)
- 4: Medical facility-side information processing terminal (e.g., PC)
- 10: Server
- 10A1: Diagnostic cross-sectional assessment part
- 10A2: Disease-specific assessment part
- 10B: Module selection part
- 10C: Medical treatment module part
- 10D: Evaluation part
- 10F: Database
- 10H: Login/authentication part
- 100: System

## Claims

1. A system comprising a server operates a control unit and an information processing terminal used for a patient, wherein the server comprises:
a block having a function for assessing a degree how a patient is adaptively behaving or non-adaptively behaving against to psychosocial issues, lifestyle adaptation, stress, and the like by using normal lifestyle data, and/or, a block having a function for assessing the degree how the patient is adaptively behaving or non-adaptively behaving against to the characteristic symptoms of the disease; and
a block having a function for analyzing results of the assessments of the blocks, for classifying the patient into a specific group, and for selecting a medical treatment module corresponding to the classified group.

2. A system comprising a server operates as a control unit and an information processing terminal used for a patient, wherein the server comprises a block having a function for assessing a degree how a patient is adaptively behaving or non-adaptively behaving against to psychosocial issues, lifestyle adaptation, stress and the like by using normal lifestyle data;
or wherein the server comprises a block having a function for assessing a degree how a patient is adaptively behaving or non-adaptively behaving against to psychosocial issues, lifestyle adaptation, stress and the like by using normal lifestyle data, and, a block having a function for assessing the degree how the patient is adaptively behaving or non-adaptively behaving against to the characteristic symptoms of the disease.

3. The system according to claims 1 or 2, comprising a device for obtaining normal lifestyle data.

4. The system according to any one of claims 1-3, further comprising an information processing terminal used in a medical facility side.

5. The system according to any one of claims 1-4, wherein the system is used in conjunction with regenerative medicine using stem cells or a medical treatment using culture supernatants of stem cells.

6. The system according to any one of claims 1-5, wherein the system is applied to a medical treatment of any disease of issues of relationship with a spouse or a partner, erectile dysfunction, depression, obsessive-compulsive disorder, panic disorder, agoraphobia, generalized anxiety, social anxiety and speech anxiety, specific phobic disorder, post-traumatic stress disorder, insomnia, irritable bowel syndrome, eating disorder and obesity, obesity and other lifestyle-related diseases, bipolar disorder, borderline personality disorder, attention-deficit / hyperactivity disorder, chronicity or persistent pain, sexual dysfunction, issues of relationship with a spouse or a partner except for erectile dysfunction, alcoholism and other addictions, schizophrenia and other severe psychoses, infertility, atopic dermatitis, an overactive bladder, disease related to anti-aging, menopausal disorder, psychogenic visual disturbance, dental psychosomatic disease, dysmorphic phobia and premenstrual syndrome.

7. A method comprising a step for assessing a degree how a patient is adaptively behaving or non-adaptively behaving against to psychosocial issues, lifestyle adaptation, by using normal lifestyle data, and
steps for analyzing results of the assessments, for classifying a patient into a specific group, and for selecting a medical treatment module corresponding to the classified groups.

8. The method according to claim 7, comprising a step for assessing the degree how the patient is adaptively behaving or non-adaptively behaving against to the characteristic symptoms of the disease,
In said step for selecting, a patient is classified by analyzing a result of assessment whether a skill is demonstrated for a specified disease.

9. The method according to any one of claims 1-5, wherein the method is applied to a medical treatment of any disease of issues of relationship with a spouse or a partner, erectile dysfunction, depression, obsessive-compulsive disorder, panic disorder, agoraphobia, generalized anxiety, social anxiety and speech anxiety, specific phobic disorder, post-traumatic stress disorder, insomnia, irritable bowel syndrome, eating disorder and obesity, obesity and other lifestyle-related diseases, bipolar disorder, borderline personality disorder, attention-deficit / hyperactivity disorder, chronicity or persistent pain, sexual dysfunction, issues of relationship with a spouse or a partner except for erectile dysfunction, alcoholism and other addictions, schizophrenia and other severe psychoses, infertility, atopic dermatitis, an overactive bladder, disease related to anti-aging, menopausal disorder, psychogenic visual disturbance, dental psychosomatic disease, dysmorphic phobia and premenstrual syndrome.
